# EUROPEAN PATENT APPLICATION

(11) **EP 4 230 221 A1**
(43) Date of publication of application: **23.08.2023**
(21) Application number: 21880494.6
(22) Date of filing: 13.10.2021
(51) Int. Cl.: A61K 38/17, A61K 9/00, A61P 27/02, C07K 14/47, C12N 15/85

(54) **PHARMACEUTICAL COMPOSITION FOR PREVENTION OR TREATMENT OF RETINAL OR CHOROIDAL DISEASE COMPRISING PLVAP AS ACTIVE INGREDIENT**

(30) Priority: 13.10.2020 KR 20200131953
(71) Applicant: The Asan Foundation, Seoul 05505 (KR); University Of Ulsan Foundation For Industry Cooperation, Ulsan 44610 (KR)
(72) Inventor: LEE, Junyeop, Seoul 05555 (KR); KIM, Soo Jin, Daegu 42471 (KR)
(74) Representative: Dehns
(86) International application number: PCT/KR2021/014090
(87) International publication number: WO 2022/080847

(57) **Abstract**

The present invention relates to a pharmaceutical composition for the prevention or treatment of a retinal disease, including PLVAP as an active ingredient, and, on the basis that a reduction in PLVAP may lead to retinal degeneration by causing a change in the ultra-microstructure of choroidal vessels, the present inventors confirmed that the administration of PLVAP plasmid DNA to an animal model of a retinal disease had the effect of increasing fenestrations, which is important for maintaining the normal function of choroidal vessels, while maintaining the structure and function of choroidal vessels. Therefore, the PLVAP protein or gene is expected to be usefully used for the treatment of a retinal or choroidal disease.

## Description

### [Technical Field]

The present invention relates to a pharmaceutical composition for the prevention or treatment of a retinal or choroidal disease, comprising PLVAP as an active ingredient, and the like.

This application claims priority to and the benefit of Korean Patent Application No. 10-2020-0131953 filed in the Korean Intellectual Property Office on October 13, 2020, and all the contents disclosed in the specification and drawings of that application are incorporated in this application.

### [Background Art]

The nerve tissue located in the center of the inner retina of the eye is called the macula, and since most of the visual cells responding to a light stimulus are gathered in the macula and a place where an image of an object is formed is also at the center of the macula, the macula plays a very important role in vision. Age-related macular degeneration (AMD) is a chronic disease characterized by degeneration of the retinal pigment epithelium, Bruch's membrane, and choroidal capillaries of the macula. Anatomically, the neurosensory retina is located in front of the retinal pigment epithelium and depends on the retinal pigment epithelium for its nutrition, support, recirculation, and waste disposal. The Bruch's membrane is a five-layered structure, and is interposed between the choroid and the retinal pigment epithelium. The innermost layer is the basement membrane of the retinal pigment epithelium and the outermost layer is the basement membrane of the choroidal capillary endothelial cells. That is, AMD is a degenerative disease that develops in the retinal pigment epithelium, Bruch's membrane and choroidal capillary complex.

This disease mainly occurs in people aged 50 years or older, and in the West, this disease is already the main cause of blindness in people aged 60 years or older, and it also tends to be increasing in Korea. Although the cause of AMD is not clearly understood yet, there are age (which exhibits a sharp increase particularly after the age of 75), hypertension, obesity, genetic predisposition, excessive UV exposure, low serum antioxidant concentration, and the like, in addition to smoking which is an environmental factor that have received the most attention, as the known risk factors.

There are two types of macular degeneration, which are dry (nonexudative) macular degeneration and wet (exudative) macular degeneration. In the case of dry macular degeneration (dry AMD, nonexudative AMD, nonneovascular AMD), waste products form a yellow deposit called drusen under the retina and when this deposit accumulates, it disturbs blood flow to the retina, particularly to the macula, resulting in blurry vision and visual impairment. Although dry macular degeneration does not cause a sudden loss of vision, it may progress to wet macular degeneration (wet AMD, exudative AMD, neovascular AMD). Below the retina, vascular aggregations embedded within fibrous tissue is caused by the growth of new blood vessels in the choroidal area below the retina. When the weak new blood vessel is broken, it results in bleeding or exudation, which causes degeneration of the macular region of the retina to cause vision impairment. It is known that wet macular degeneration progresses rapidly, leading to rapid deterioration of vision within several weeks and blindness within two months to three years.

Meanwhile, as known treatment methods for macular degeneration to date, photodynamic therapy (PDT) and anti-vascular endothelial growth factor antibody (anti-VEGF) injection have been used. PDT is a method of selectively destroying new blood vessels by irradiating the eyes with a special laser only reacting to a light-sensitive material when visudyene, which is the light-sensitive material, is injected through a blood vessel and then reaches the new blood vessels of the retina after a predetermined time. However, there are many cases of recurrence even after treatment, so that there are disadvantages in that the treatment must be repeated in many cases, and the retina itself may also be damaged.

Anti-VEGF injection is a method (intravitreal injection) of directly injecting an antibody (anti-VEGF) that inhibits the formation and proliferation of new blood vessels by selectively binding to vascular endothelial growth factor (VEGF), which is an important factor for the generation and progression of new blood vessels. Examples of the protein antibodies used for anti-VEGF injection include Lucentis (ranibizumab), Eylea (aflibercept), Beovu (brolucizumab), and Avastin (bevacizumab), Lucentis, Eylea, and Beovu are drugs approved by the FDA as therapeutic agents for wet macular degeneration, and Avastin is a drug approved for the treatment of cancer and is used for eye diseases. However, the protein treatment method is expensive, and a drug is directly injected into the eye because the drug cannot be dropped or applied to the eye, and needs to be periodically administered about once every 1 to 3 months, so there is a risk of bleeding, infection, retinal detachment, and the like.

In addition, choroidal vessels serve to supply oxygen and nutrients to the retina through the fenestrations of endothelial cells and discharge metabolites generated in the retina into the choroid, and the fenestrations of the choroidal vessels are mostly directed towards the retina to help excrete waste. However, although anti-vascular endothelial growth factor antibody treatment effectively suppresses angiogenesis, research results have reported that the treatment rather exacerbates retinal degeneration by reducing fenestrations, which is important for maintaining the normal function of the choroidal vessels. Therefore, there is a need for developing a new technique of treating the disease while maintaining the ultra-microstructure and function of the choroidal vessels.

Diabetic retinopathy is the most common cause of blindness in young people and is a vascular complication caused by chronic hyperglycemia. The occurrence of such diabetic retinopathy induces the closure of capillaries due to the loss of perivascular cells, and retinal hemorrhage and detachment due to the proliferation of new blood vessels to induce blindness by causing the loss of function of retinal nerves. Although antibody injection therapy that suppresses, vascular endothelial growth factor (VEGF), including Lucentis, has been used to treat diabetic retinopathy, repeated injections are needed, and it is difficult to restore the fundamental vasculature and function. Diabetic chorioretinopathy is a disease that may occur in the presence or absence of such diabetic retinopathy, and is characterized by the expansion of choroidal vessels, an increase in the leakage of choroidal vessels, and the inflammation and ischemia of choroidal tissue. Such choroidal changes caused by diabetes lead to degeneration of the retinal pigment epithelium, thereby leading to loss of visual cells and deterioration of vision. There is a lack of research on the etiology and treatment targets of diabetic chorioretinopathy, so there is a need for developing a new technique because there is no effective therapeutic agent capable of recovering from or preventing diabetic chorioretinopathy.

Furthermore, no attempts have been made to change the ultra-microstructure of choroidal vessels for the treatment of retinal or choroidal diseases including age-related macular degeneration and diabetic chorioretinopathy.

### [Disclosure]

### [Technical Problem]

Thus, as a result of constructing a recombinant plasmid DNA expressing PLVAP and subretinally injecting the recombinant plasmid into mouse models of age-related macular degeneration and diabetic chorioretinopathy, the present inventors confirmed through the supplementation of PLVAP that not only the ultrastructural polarity of choroidal vessels can be normalized, but also the effect of excreting waste is significant by restoring the structure and function of endothelial cell fenestrations, and as a result, the effects of improving the retinal structure and improving visual function occur, thereby completing the present invention.

Therefore, an object of the present invention is to provide a pharmaceutical composition for preventing or treating a retinal or choroidal disease, the pharmaceutical composition comprising a plasmalemma vesicle-associated protein (PLVAP) protein, an expression or activity activator of the protein, or a nucleotide sequence encoding the PLVAP protein, as an active ingredient.

Another object of the present invention is to provide a quasi-drug composition for the prevention or treatment of a retinal or choroidal disease, the quasi-drug composition comprising a plasmalemma vesicle-associated protein (PLVAP) protein, an expression or activity activator of the protein, or a nucleotide sequence encoding the PLVAP protein, as an active ingredient.

Still another object of the present invention is to provide a pharmaceutical composition for improving un ultra-microstructure of choroidal vessels, comprising a plasmalemma vesicle-associated protein (PLVAP) protein, an expression or activity activator of the protein, or an isolated nucleotide sequence encoding the PLVAP protein as an active ingredient.

However, the technical problems to be achieved by the present invention are not limited to the aforementioned problems, and other problems that are not mentioned may be clearly understood by those skilled in the art from the following description.

### [Technical Solution]

To achieve the objects, the present invention provides a pharmaceutical composition for preventing or treating a retinal or choroidal disease, the pharmaceutical composition comprising a plasmalemma vesicle-associated protein (PLVAP) protein, an expression or activity activator of the protein, or a nucleotide sequence encoding the PLVAP protein, as an active ingredient.

In an exemplary embodiment of the present invention, the PLVAP protein may comprise an amino acid sequence represented by SEQ ID NO: 25, or an amino acid sequence having a sequence homology of 80% or more therewith, but is not limited thereto.

In another exemplary embodiment of the present invention, the PVLAP protein may be a PLVAP protein, a fragment thereof, a variant thereof, or a fusion protein thereof, but is not limited thereto.

In still another exemplary embodiment of the present invention, a nucleotide sequence encoding the PLVAP protein may be comprised in a recombinant vector, but is not limited thereto.

The recombinant vector may comprise a promoter, an enhancer, an intron, a reporter marker sequence, a protein purification tag, a termination sequence, an mRNA stabilization sequence, an exogenous gene sequence, a cell selection marker sequence, or a combination thereof, but is not limited thereto.

In yet another exemplary embodiment of the present invention, the recombinant vector comprises a promoter that induces specific expression in vascular endothelial cells, and the promoter may be one or more selected from the group consisting of CD144, TIE1, FLT1, and EMCN, but is not limited thereto.

In yet another exemplary embodiment of the present invention, the retinal or choroidal disease may be a retinal or choroidal disease associated with vasopermeability, but is not limited thereto.

In yet another exemplary embodiment of the present invention, the retinal or choroidal disease may be retinitis pigmentosa (RP), Leber congenital amaurosis (LCA), Stargardt disease, Usher's syndrome, choroideremia, rod-cone or cone-rod dystrophy, ciliopathy, a mitochondrial disorder, progressive retinal atrophy, a degenerative retinal disease, age-related macular degeneration (AMD), wet AMD, dry AMD, central serous chorioretinopathy, the pachychoroid disease spectrum, degenerative myopia, nodular choroidopathy, chorioretinitis, choroidal tumors, choroidal neovascularization, hereditary choroidal disease, geographic atrophy, familial or acquired maculopathy, a retinal photoreceptor disease, a retinal pigment epithelial-based disease, diabetic retinopathy, diabetic chorioretinopathy, cystoid macular edema, uveitis, retinal detachment, traumatic retinal injury, iatrogenic retinal injury, macular holes, macular telangiectasia, a ganglion cell disease, an optic nerve cell disease, glaucoma, optic neuropathy, an ischemic retinal disease, retinopathy of prematurity, retinal vascular occlusion, a familial retinal arterial macroaneurysm, a retinal vascular disease, an ocular vascular disease, retinal nerve cell degeneration retinal nerve cell degeneration due to glaucoma, ischemic optic neuropathy, or a combination thereof, but is not limited thereto.

In yet another exemplary embodiment of the present invention, the nucleotide sequence may comprise a base sequence represented by SEQ ID NO: 1, or a base sequence having a homology of 80% or more therewith, but is not limited thereto.

In yet another exemplary embodiment of the present invention, the pharmaceutical composition may be formulated for administering to a subject via one or more routes selected from the group consisting of oral, subcutaneous, intraperitoneal, intrapulmonary, intranasal, intramuscular, intravenous, intraarterial and topical ocular administration, but is not limited thereto.

In yet another exemplary embodiment of the present invention, the topical ocular administration may be intraconjunctival administration, intravitreal administration, subretinal administration, suprachoroidal administration, subconjunctival administration, administration under the Tenon's capsule, or a combination thereof, but is not limited thereto.

In yet another exemplary embodiment of the present invention, the pharmaceutical composition may further comprise one or more selected from the group consisting of pharmaceutically acceptable carriers, excipients and dilulents, but is not limited thereto.

In yet another exemplary embodiment of the present invention, the pharmaceutical composition may restore the fenestrations of endothelial cells, but is not limited thereto.

In yet another exemplary embodiment of the present invention, the pharmaceutical composition may further comprise an anti-VEGF agent, but is not limited thereto.

In yet another exemplary embodiment of the present invention, the pharmaceutical composition may be administered simultaneously or sequentially with the anti-VEGF agent, but is not limited thereto.

Further, the present invention provides a quasi-drug composition for the prevention or treatment of a retinal or choroidal disease, the quasi-drug composition comprising a plasmalemma vesicle-associated protein (PLVAP) protein, an expression or activity activator of the protein, or a nucleotide sequence encoding the PLVAP protein, as an active ingredient.

In addition, the present invention provides a method of preventing, ameliorating or treating a retinal or choroidal disease, the method comprising: administering a pharmaceutical composition comprising a plasmalemma vesicle-associated protein (PLVAP) protein, an expression or activity activator of the protein, or a nucleotide sequence encoding the PLVAP protein, as an active ingredient, to a subject.

In an exemplary embodiment of the present invention, the subject may be a subject in need of treatment of a retinal or choroidal disease, but is not limited thereto.

In an exemplary embodiment of the present invention, the pharmaceutical composition may be administered simultaneously or sequentially with an anti-VEGF agent, but is not limited thereto.

Furthermore, the present invention provides a use of a pharmaceutical composition comprising a plasmalemma vesicle-associated protein (PLVAP) protein, an expression or activity activator of the protein, or a nucleotide sequence encoding the PLVAP protein as an active ingredient for the prevention, amelioration or treatment of a retinal or choroidal disease.

Further, the present invention provides a use of a plasmalemma vesicle-associated protein (PLVAP) protein, an expression or activity activator of the protein, or a nucleotide sequence encoding the PLVAP protein for producing a drug for the prevention or treatment of a retinal or choroidal disease.

In addition, the present invention provides a plasmalemma vesicle-associated protein (PLVAP) protein, an expression or activity activator of the protein, or a nucleotide sequence encoding the PLVAP protein, which is characterized by improving the ultra-microstructure of choroidal vessels.

Furthermore, the present invention provides a pharmaceutical composition for improving an ultra-microstructure of choroidal vessels, the pharmaceutical composition comprising a plasmalemma vesicle-associated protein (PLVAP) protein, an expression or activity activator of the protein, or an isolated nucleotide sequence encoding the PLVAP protein, as an active ingredient.

Further, the present invention provides a method of improving an ultra-microstructure of choroidal vessels, the method comprising: administering a composition comprising a plasmalemma vesicle-associated protein (PLVAP) protein, an expression or activity activator of the protein, or an isolated nucleotide sequence encoding the PLVAP protein, as an active ingredient, to a subject.

In addition, the present invention provides a use of a composition comprising a plasmalemma vesicle-associated protein (PLVAP) protein, an expression or activity activator of the protein, or an isolated nucleotide sequence encoding the PLVAP protein as an active ingredient for the improvement of the ultra-microstructure of choroidal vessels.

### [Advantageous Effects]

On the basis that a reduction in PLVAP may lead to retinal degeneration by causing a change in the ultra-microstructure of choroidal vessels, the present inventors confirmed that the administration of PLVAP plasmid DNA to an animal model of a retinal or choroidal disease had the effect of increasing fenestrations, which is important for maintaining the normal function of choroidal vessels, while maintaining the structure and function of choroidal vessels. Therefore, the PLVAP protein or gene is expected to be usefully used for the treatment of a retinal or choroidal disease.

### [Description of Drawings]

FIG. 1 illustrates the structure and constituent elements of PLVAP plasmid DNA according to an exemplary embodiment of the present invention.
FIG. 2 illustrates the expression of PLVAP as a result of treating human choroidal endothelial cells with PLVAP plasmid DNA according to an exemplary embodiment of the present invention according to the type of promoter and integrase.
FIG. 3 illustrates the expression rate of PLVAP 72 hours after subretinal injection of PLVAP plasmid DNA according to an exemplary embodiment of the present invention into mice according to the type of promoter.
FIG. 4 illustrates the results of confirming the deformation of the ultra-microstructure of choroidal vessels in a mouse model of age-related macular degeneration according to an exemplary embodiment of the present invention.
FIG. 5 illustrates the number of fenestrations in choroidal vessels and the number of disoriented fenestrations in a mouse model of dry age-related macular degeneration according to an exemplary embodiment of the present invention.
FIG. 6 illustrates the results of confirming the excretion pattern of retinal metabolic waste in the subretinal space and Bruch's membrane in a mouse model of dry age-related macular degeneration according to an exemplary embodiment of the present invention.
FIG. 7 illustrates the results of confirming a pattern in which the number of fenestrations in choroidal blood vessels increased and the diaphragm was restored as a result of administering PLVAP plasmid DNA to a mouse model of dry age-related macular degeneration according to an exemplary embodiment of the present invention.
FIGS. 8A and 8B illustrate the result of confirming that lipid metabolites were well excreted and lipid droplets and accumulation were almost completely lost as a result of administering PLVAP plasmid DNA to a mouse model of dry age-related macular degeneration according to an exemplary embodiment of the present invention.
FIGS. 9A and 9B illustrate the results of confirming the degree of progression of macular degeneration as a result of administering PLVAP plasmid DNA to a mouse model of dry age-related macular degeneration according to an exemplary embodiment of the present invention by fundus photography.
FIGS. 10A and 10B illustrate the results of confirming the degree of progression of macular degeneration as a result of administering PLVAP plasmid DNA to a mouse model of dry age-related macular degeneration according to an exemplary embodiment of the present invention by fundus photography and choroidal angiography.
FIG. 11 illustrates the results of confirming that deteriorated visual function was restored as a result of administering PLVAP plasmid DNA to a mouse model of dry age-related macular degeneration according to an exemplary embodiment of the present invention by an electroretinogram.
FIG. 12 illustrates the results of confirming that choroidal neovascularization was suppressed as a result of administering PLVAP plasmid DNA to a mouse model of wet age-related macular degeneration according to an exemplary embodiment of the present invention.
FIG. 13 illustrates the results of confirming the deformation of the ultra-microstructure of choroidal vessels in a mouse model of diabetic chorioretinopathy according to an exemplary embodiment of the present invention.
FIGS. 14A and 14B illustrate the number of fenestrations of choroidal vessels, the number of disoriented fenestrations, and the proportion of abnormal fenestrations in which the diaphragm was lost in mouse models of diabetic chorioretinopathy according to an exemplary embodiment of the present invention.
FIG. 15 illustrates the results of confirming that the number of fenestrations of choroidal vessels increased as a result of administering PLVAP plasmid DNA to a mouse model of diabetic chorioretinopathy according to an exemplary embodiment of the present invention.
FIG. 16 illustrates the results of confirming the degree of progression of macular degeneration as a result of administering PLVAP plasmid DNA to a mouse model of diabetic chorioretinopathy according to an exemplary embodiment of the present invention by fundus photography.
FIG. 17 illustrates the results of confirming that deteriorated visual function was restored as a result of administering PLVAP plasmid DNA to a mouse model of diabetic chorioretinopathy according to an exemplary embodiment of the present invention by an electroretinogram.
FIG. 18 illustrates the structure and constituent elements of a PLVAP mammal plasmid vector according to an exemplary embodiment of the present invention.
FIGS. 19A and 19B illustrate the results of confirming that the number of fenestrations of choroidal vessels increased and the polarity of choroidal vessels was normalized as a result of administering PLVAP plasmid DNA to a mouse model of PLVAP^{ECKO} according to an exemplary embodiment of the present invention.
FIG. 20 illustrates the change in ultra-microstructure of choroidal vessels of age-related macular degeneration/diabetic chorioretinopathy after the injection of the PLVAP plasmid according to an exemplary embodiment of the present invention.
FIGS. 21A to 21E are the results of confirming the polarity of choroidal vessels according to an exemplary embodiment of the present invention, confirming that PLVAP was reduced by aging or diabetes, and the ultra-microstructure of choroidal vessels was changed by the reduction in PLVAP.

### [Best Modes]

The present invention relates to a pharmaceutical composition for preventing or treating a retinal or choroidal disease, the pharmaceutical composition comprising a plasmalemma vesicle-associated protein (PLVAP) protein, an expression or activity activator of the protein, or a nucleotide sequence encoding the PLVAP protein, as an active ingredient.

Furthermore, the present invention relates to a plasmalemma vesicle-associated protein (PLVAP) protein, an expression or activity activator of the protein, or a nucleotide sequence encoding the PLVAP protein, which is characterized by improving the ultra-microstructure of choroidal vessels.

Hereinafter, the present invention will be described in detail.

In the present invention, PLVAP is also named plasmalemma vesicle-associated protein, or PV-1, and refers to a naturally occurring or endogenous PLVAP (for example, mammalian, human) protein, and a protein having an amino acid sequence that is the same or substantially the same as that of a naturally occurring or endogenous PLVAP protein (for example, a recombinant protein, synthetic protein). Accordingly, the term PLVAP, which is used interchangeably herein, comprises a polymorphic or allelic variant and other isoforms of a PLVAP protein produced by, for example, alternative splicing or other cellular processes, which occurs naturally in mammals (for example, humans). In the present invention, the PLVAP protein may comprise or consist of an amino acid sequence of NCBI Reference Sequence: NP_112600. 1, but is not limited thereto.

In the present invention, a nucleotide sequence encoding the PLVAP protein may comprise a base sequence represented by SEQ ID NO: 1, but is not limited thereto. As used herein, a "nucleotide sequence encoding a PLVAP protein" may comprise a nucleotide sequence represented by SEQ ID NO:1, and preferably may consist of the nucleotide sequence represented by SEQ ID NO:1. Further, the nucleotide variant is comprised in the scope of the present invention. Specifically, the nucleotide sequence may comprise a nucleotide sequence having a sequence homology of 80% or more, preferably 90% or more with a base sequence of SEQ ID NO: 1. For example, the nucleotide sequence comprises a polynucleotide having a sequence homology of 80%, 81%, 82%, 83%, 84%, 85%, 86%, 87%, 88%, 89%, 90%, 91%, 92%, 93%, 94%, 95%, 96%, 97%, 98%, 99%, or 100%.

In the present invention, the PLVAP protein may comprise an amino acid sequence represented by SEQ ID NO: 25, but is not limited thereto. As used herein, a "PLVAP protein" may comprise an amino acid sequence represented by SEQ ID NO: 25, and preferably may consist of a base sequence represented by SEQ ID NO: 25. In addition, the PLVAP protein, a fragment thereof, a variant thereof, or a fusion protein thereof is comprised in the scope of the present invention. Specifically, the protein may comprise an amino acid sequence having a sequence homology of 80% or more, preferably 90% or more with an amino acid sequence of SEQ ID NO: 25. For example, the protein comprises a protein having a sequence homology of 80%, 81%, 82%, 83%, 84%, 85%, 86%, 87%, 88%, 89%, 90%, 91%, 92%, 93%, 94%, 95%, 96%, 97%, 98%, 99%, and 100%.

In the present invention, a nucleotide sequence encoding the PLVAP protein may be comprised in a recombinant vector, but is not limited thereto.

In the present invention, an "expression vector" refers to a vector capable of expressing a peptide or protein encoded by a foreign nucleic acid inserted in the vector, preferably a recombinant expression vector prepared so as to express a PLVAP protein. The "vector" refers to any medium for the introduction and/or transfer of a base in a host cell in vitro, ex vivo, or in vivo, and may be a replicon to which another DNA fragment may be bound to bring about the replication of the bound fragment, and the "replicon" refers to any genetic unit (for example, a plasmid, a phage, a cosmid, a chromosome, a virus, and the like) that functions as an autonomous unit of DNA replication in vivo, that is, one which is capable of replication under its own control. The recombinant expression vector of the present may preferably comprise a promoter which is a transcription initiation factor to which RNA polymerase binds, any operator sequence for regulating transcription, a sequence encoding a suitable mRNA ribosome binding site and a sequence for regulating the termination of transcription and translation, a terminator, and the like, and may also further comprise a selection marker gene such as an antibiotic resistance gene for selecting a transformant, and the like.

In the present invention, a nucleotide sequence encoding a PLVAP protein may be modified by substitution, deletion, insertion or a combination thereof of one or more nucleic acid bases as long as it encodes a protein having activity equivalent to that of PLVAP. The sequence of such a nucleic acid molecule may be singlestranded or double-stranded, and may be a DNA molecule or a RNA (mRNA) molecule.

In the present invention, the vector comprises a liposome, a plasmid vector, a cosmid vector, a bacteriophage vector, a viral vector, and the like, but is not limited thereto. In the present invention, examples of a preferred viral vector comprise adenovirus, adeno-associated virus, retrovirus, lentivirus, herpes simplex virus, alpha virus, and the like. In the present invention, the vector may comprise a promoter, an enhancer, an intron, a reporter marker sequence, a protein purification tag, a termination sequence, an mRNA stabilization sequence, an exogenous gene sequence, a cell selection marker sequence, or a combination thereof. The recombinant vector of the invention may comprise a nucleic acid encoding PLVAP and a regulatory sequence for the transcription or translation thereof. In particular, an important regulatory sequence regulates the transcription initiation like a promoter and an enhancer. Furthermore, the important regulatory sequence may comprise a regulatory sequence consisting of an initiation codon, a termination codon, a polyadenylation signal, a Kozak sequence, an enhancer, a signal sequence for membrane targeting and secretion, an internal ribosome entry site (IRES), and the like. Such a regulatory sequence and a nucleic acid encoding PLVAP need to be operably linked.

In the present invention, the enhancer may include a CMV enhancer, an RSV enhancer, an alpha fetoprotein enhancer, a TTR minimal promoter/enhancer, TH-binding globulin promoter/alpha-microglobulin/bikunin enhancer (LSP), an APB enhancer, an ABPS enhancer, an alpha mic/bik enhancer, a TTR enhancer, en34, or ApoE, but is not limited thereto.

In the present invention, the intron may be selected from among CBA, human beta globin, IVS2, SV40, bGH, alpha-globulin, beta-globulin, collagen, ovalbumin, p53, or a fragment thereof, but is not limited thereto.

In the present invention, the reporter marker sequence comprises various known reporter marker sequences, and may be preferably a green fluorescent protein (GFP) gene, an eGFP gene, a luciferase gene, a β-galactosidase gene, a chloramphenicol acetyltransferase gene, a human growth hormone gene or a secreted placental alkaline phosphatase gene, but is not limited thereto.

In the present invention, a nucleotide sequence encoding the PLVAP protein of the present invention may be administered in a form in which a recombinant DNA molecule comprising the polynucleotide is operably linked to a nucleic acid sequence regulating expression, for example, in the form of an expression vector, such that the recombinant DNA molecule is expressed in a patient to be treated. It is preferred that the vector comprises a suitable transcription regulatory signal comprising a promoter site capable of expressing a coding sequence, and the promoter is operable in a patient to be treated. Therefore, a promoter, which is a term encompassing not only a sequence required to direct RNA polymerase to a transcription initiation site for human gene therapy, but also other operational or regulatory sequences comprising an enhancer, if appropriate, may comprise a pEMU promoter, an MAS promoter, a histone promoter, a Clp promoter, a cauliflower mosaic virus-derived 35S promoter, a cauliflower mosaic virus-derived 19S RNA promoter, a plant actin protein promoter, a ubiqitin protein promoter, a cytomegalovirus (CMV) promoter, a simian virus 40 (SV40) promoter, a respiratory syncytial virus (RSV) promoter, an elongation factor-1 alpha (EF-1α) promoter, and the like, and may be preferably a human promoter sequence from a human gene or a human promoter sequence from a gene commonly expressed in humans, for example, a promoter from human CMV

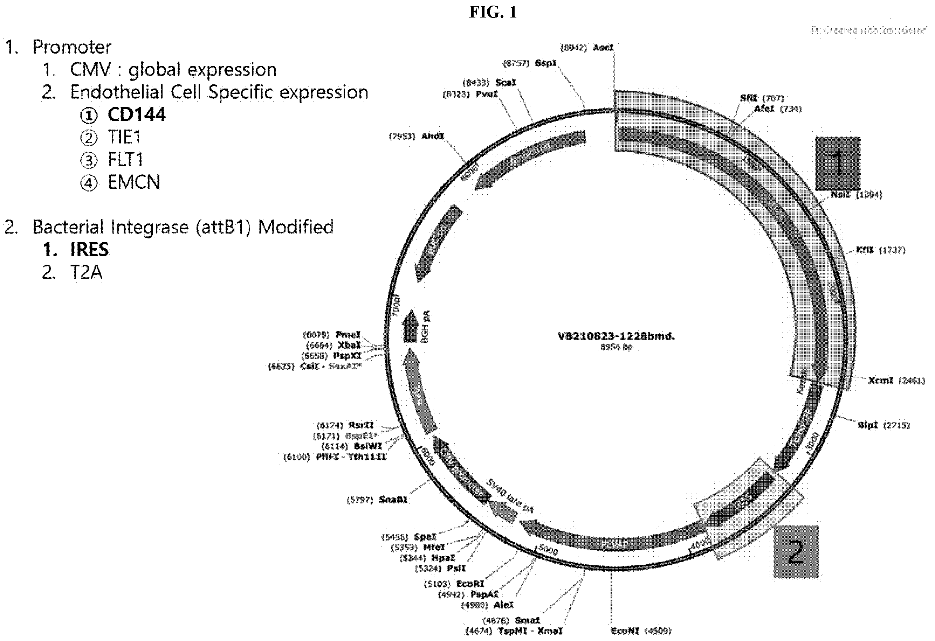

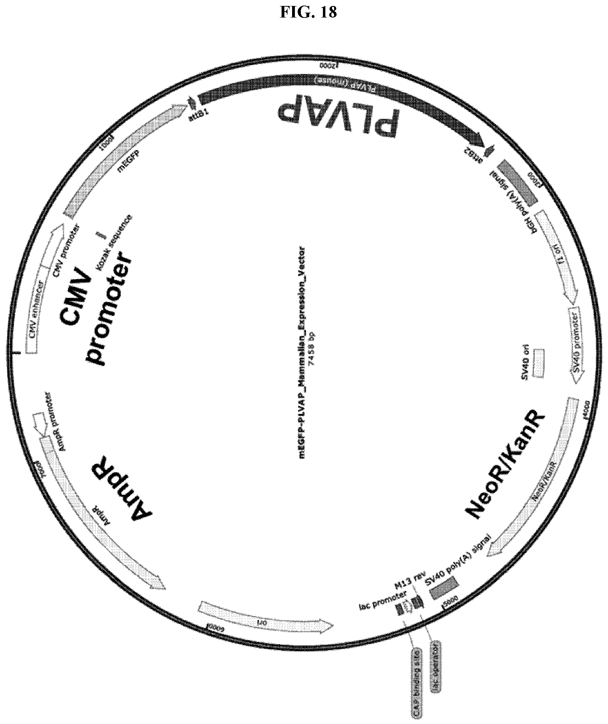

In the present invention, the vector may be represented by the cleavage map disclosed in FIG. 1 or FIG. 18, but is not limited thereto.

In the present invention, the recombinant vector may further comprise a promoter specifically expressed in one or more vascular endothelial cells selected from the group consisting of CD144, TIE1, FLT1, and EMCN, and may preferably comprise CD144, but is not limited thereto.

In the present invention, the recombinant vector may comprise an internal ribosome entry site (IRES) or a 2A peptide, but is not limited thereto.

In the present invention, "A peptide" refers to a small (18-22 amino acids) peptide sequence that allows efficient concordant expression of individual protein products in a single coding sequence. Any convenient 2A peptide, for example, a 2A peptide from viruses, such as foot-and-mouth disease virus (F2A), equine Rhinitis A virus, porcine teschovirus-1(P2A) or Thosea asigna virus (T2A), or any one among 2A peptides described in Szymczak-Workman, A. et al. "Design and Construction of 2A Peptide-Linked Multicistronic Vectors" may be used in the targeting vector. Document [Gene Transfer: Delivery and Expression of DNA and RNA (ed. Friedmann and Rossi). CSHL Press, Cold Spring Harbor, NY, USA, 2007], the disclosure of which is incorporated herein by reference.

In the present invention, the retinal or choroidal disease may be a vascular permeability-related retinal or choroidal disease, but is not limited thereto. The "vascular permeability-related retinal or choroidal disease" is a retinal disease that results from disruption of the normal regulation of vascular permeability, and generally refers to a retinal or choroidal disease causing bleeding, edema and inflammation due to an increase in permeability caused by changes in blood vessels.

In the present invention, the retinal or choroidal disease may be one or more selected from the group consisting of retinitis pigmentosa (RP), Leber congenital amaurosis (LCA), Stargardt disease, Usher's syndrome, choroideremia, rod-cone or cone-rod dystrophy, ciliopathy, a mitochondrial disorder, progressive retinal atrophy, a degenerative retinal disease, age-related macular degeneration (AMD), wet AMD, dry AMD, central serous chorioretinopathy, the pachychoroid disease spectrum, degenerative myopia, nodular choroidopathy, chorioretinitis, choroidal tumors, choroidal neovascularization, hereditary choroidal disease, geographic atrophy, familial or acquired maculopathy, a retinal photoreceptor disease, a retinal pigment epithelial-based disease, diabetic retinopathy, diabetic chorioretinopathy, cystoid macular edema, uveitis, retinal detachment, traumatic retinal injury, iatrogenic retinal injury, macular holes, macular telangiectasia, a ganglion cell disease, an optic nerve cell disease, glaucoma, optic neuropathy, an ischemic retinal disease, retinopathy of prematurity, retinal vascular occlusion, a familial retinal arterial macroaneurysm, a retinal vascular disease, an ocular vascular disease, retinal nerve cell degeneration retinal nerve cell degeneration due to glaucoma, and ischemic optic neuropathy, but is not limited thereto.

In the present invention, "age-related macular degeneration (AMD)" is a disease caused by various changes that occur with aging in the macula of the retina, which plays a very important role in vision. In the present invention, the age-related macular degeneration may include dry (nonexudative) AMD or wet (exudative) AMD. "Dry AMD" refers to cases in which lesions such as drusen or retinal pigment epithelium atrophy occur in the retina, and accounts for nearly 90% of AMD. As the visual cells present in the macula gradually atrophy, the vision gradually deteriorates over time, and the AMD may develop into a wet form. In "wet AMD", choroidal neovascularization grows under the retina, and this neovascularization itself or bleeding, exudation, and the like from the blood vessels is or are likely to cause severe visual impairment, and may lead to blindness due to disc-shaped atrophy, severe bleeding, and the like within several months to several years after the onset of the disease.

In the present invention, "diabetic retinopathy" is one of the microvascular complications that occur in diabetes, and occurs due to the functional and morphological changes of capillaries, such as ischemia with increased vascular permeability and neovascularization of the retina due to hyperglycemia and various biochemical changes accompanying the same. In the present invention, the diabetic retinopathy may comprise non-proliferative or proliferative retinopathy, but is not limited thereto. "Non-proliferative retinopathy" refers to a condition in which the small blood vessels of the retina weaken to leak serum or become clogged, thereby interrupting nutritional supply. It is known that "proliferative retinopathy" is caused by the formation of new blood vessels in areas of poor blood circulation, and bleeding occurring from the new blood vessels leads to blindness within 5 years unless appropriate treatment is given.

In the present invention, "diabetic chorioretinopathy (diabetic choroidopathy)" is one of the microvascular complications that occur in the choroidal blood vessels in diabetes, and occurs as chronic hyperglycemia inhibits fenestrations and endothelial transport in the choroidal capillary layer, and the endothelial transport of macromolecules between the choroid and the retina is damaged by diabetes. Furthermore, the loss and degeneration of perivascular cells are also accompanied by structural and functional changes in large choroidal vessels such as arteries and veins, leading to inflammation and ischemia of the choroidal tissue. The diabetic chorioretinopathy may be a disease in which vision deteriorates due to the induction of structural and functional abnormalities in the choroid, which cause retinal malnutrition and the accumulation of metabolic waste.

In the present invention, "the choroid" is one of the eyeball walls located between the sclera and the retina, and is a thin membrane rich in blood vessels and melanocytes in the posterior eyeball. It is known that the choroid plays a role in preventing light incident from the outside from being scattered, and acts as the choroid at the posterior part of the eye and the iris at the anterior part of the eye.

In the present invention, "the ultra-microstructure of the choroidal vessels" may refer to one or more comprising the subretinal space, Bruch's membrane, the retinal pigment epithelium, the choroidal vessels, the endothelial cells of the choroidal vessels, the fenestrations of the endothelial cells, and the overall structure of the above components, but is not limited thereto. The ultra-microstructure may be confirmed by fundus photography, choroidal angiography, electroretinograms, and the like, but is not limited thereto.

In the present invention, "the improvement of ultra-microstructure of choroidal vessels" refers to all actions which reduce the severity of symptoms including parameters related to deformation of the choroidal vascular ultra-microstructure, for example, a decrease in the number of fenestrations, a change in the polarity of fenestrations, a change in the thickness of endothelial cells, an accumulation of retinal waste in endothelial cells, and a loss of fenestration diaphragms.

In the present invention, the pharmaceutical composition may increase or restore the fenestrations of endothelial cells, but is not limited thereto. In the present invention, the restoration of fenestrations may restore the fenestration direction of endothelial cells from the sclera side to the retina side, normalize the polarity of fenestrations toward the retina side, increase the number of fenestrations, or suppress or reduce the loss of fenestration diaphragms, but is not limited thereto.

In the present invention, the pharmaceutical composition may further comprise an anti-VEGF agent, but is not limited thereto. In the present invention, the anti-VEGF agent may be, for example, one or more selected from the group consisting of bevacizumab, ranibizumab, pegaptanib, pazopanib, sunitinib, sorafenib, regorafenib, cabozantinib, lenvatinib, ponatinib, axitinib, tivozanib, ramucirumab, vandetanib, brolucizumab, faricimab and aflibercept, but is not limited thereto. Further, the anti-VEGF agent may comprise a peptide that binds to vascular epidermal growth factors (VEGFs) to prevent or reduce the binding to the receptors thereof, an antibody that binds to VEGF, a nucleic acid capable of binding to VEGF, and the like, but is not limited thereto.

In the present invention, the pharmaceutical composition may be administered simultaneously or sequentially with the anti-VEGF agent, but is not limited thereto. In the present invention, a composition comprising the plasmalemma vesicle-associated protein (PLVAP) protein, an expression or activity activator of the protein, or a nucleotide sequence encoding the PLVAP protein as an active ingredient may be formulated so as to be present in one container in the form of a mixture with an anti-VEGF agent, and may be formulated so as to be present in separate containers and administered simultaneously or sequentially. In addition, a composition comprising the plasmalemma vesicle-associated protein (PLVAP) protein, an expression or activity activator of the protein, or a nucleotide sequence encoding the PLVAP protein as an active ingredient may be administered as secondary therapy after treatment with an anti-VEGF agent, but is not limited thereto.

The content of the plasmalemma vesicle-associated protein (PLVAP) protein, an expression or activity activator of the protein, or a nucleotide sequence encoding the PLVAP protein in the composition of the present invention can be appropriately adjusted according to the symptoms of the disease, the degree of progression of the symptoms, the condition of the patient, and the like, and may be, for example, 0.0001 to 99.9 wt%, or 0.001 to 50 wt% based on the total weight of the composition, but is not limited thereto. The content ratio is a value based on a dry amount from which the solvent is removed.

The pharmaceutical composition of the present invention may further include an appropriate carrier, excipient, and diluent, which are typically used to prepare a pharmaceutical composition. The excipient may be, for example, one or more selected from the group consisting of a diluent, a binder, a disintegrant, a lubricant, an adsorbent, a moisturizer, a film-coating material, and a controlled release additive.

The pharmaceutical composition according to the present invention may be used by being formulated into the form of a powder, a granule, a sustained-release granule, an enteric granule, a liquid, a collyrium, an elixir, an emulsion, a suspension, a spirit, a troche, aromatic water, a limonade, a tablet, a sustained-release tablet, an enteric tablet, a sublingual tablet, a hard capsule, a soft capsule, a sustained-release capsule, an enteric capsule, a pill, a tincture, a soft extract agent, a dry extract agent, a fluid extract agent, an injection, a capsule, a perfusate, an external preparation such as a plaster, a lotion, a paste, a spray, an inhalant, a patch, a sterilized injection solution, or an aerosol, and the external preparation may have a formulation such as a cream, a gel, a patch, a spray, an ointment, a plaster, a lotion, a liniment, a paste or a cataplasma.

Examples of a carrier, an excipient or a diluent which may be included in the composition according to the present invention include lactose, dextrose, sucrose, an oligosaccharide, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

When the pharmaceutical composition is prepared, the pharmaceutical composition is prepared using a diluent or excipient, such as a filler, an extender, a binder, a wetting agent, a disintegrant, and a surfactant, which are commonly used.

As an additive of the tablet, powder, granule, capsule, pill, and troche according to the present invention, it is possible to use an excipient such as corn starch, potato starch, wheat starch, lactose, sucrose, glucose, fructose, D-mannitol, precipitated calcium carbonate, synthetic aluminum silicate, calcium monohydrogen phosphate, calcium sulfate, sodium chloride, sodium hydrogen carbonate, purified lanolin, microcrystalline cellulose, dextrin, sodium alginate, methyl cellulose, carboxymethyl cellulose sodium, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and Primojel; and a binder such as gelatin, arabic gum, ethanol, agar powder, cellulose acetate phthalate, carboxymethyl cellulose, carboxymethyl cellulose calcium, glucose, purified water, sodium caseinate, glycerin, stearic acid, carboxymethyl cellulose sodium, methylcellulose sodium, methylcellulose, microcrystalline cellulose, dextrin, hydroxycellulose, hydroxypropyl starch, hydroxymethyl cellulose, purified shellac, starch, hydroxypropyl cellulose, hydroxypropyl methyl cellulose, polyvinyl alcohol, and polyvinylpyrrolidone, and it is possible to use a disintegrant such as hydroxypropyl methyl cellulose, corn starch, agar powder, methyl cellulose, bentonite, hydroxypropyl starch, carboxymethyl cellulose sodium, sodium alginate, carboxymethyl cellulose calcium, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, arabic gum, amylopectin, pectin, sodium polyphosphate, ethyl cellulose, sucrose, magnesium aluminum silicate, a D-sorbitol solution, and light anhydrous silicic acid; and a lubricant such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol 4000, PEG 6000, liquid paraffin, hydrogenated soybean oil (Lubriwax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid.

As an additive for liquid formulation according to the present invention, it is possible to use water, diluted hydrochloric acid, diluted sulfuric acid, sodium citrate, sucrose monostearate, polyoxyethylene sorbitol fatty acid esters (Tween esters), polyoxyethylene monoalkyl ether, lanolin ether, lanolin esters, acetic acid, hydrochloric acid, aqueous ammonia, ammonium carbonate, potassium hydroxide, sodium hydroxide, prolamin, polyvinyl pyrrolidone, ethyl cellulose, carboxymethyl cellulose sodium, and the like.

In a syrup according to the present invention, a solution of sucrose, other sugars or sweeteners, and the like may be used, and a fragrance, a colorant, a preservative, a stabilizer, a suspending agent, an emulsifier, a thickener, and the like may be used, if necessary.

Purified water may be used for the emulsion according to the present invention, and an emulsifier, a preservative, a stabilizer, a fragrance, and the like may be used, if necessary.

In the suspension according to the present invention, a suspending agent such as acacia, tragacanth, methyl cellulose, carboxymethyl cellulose, carboxymethyl cellulose sodium, microcrystalline cellulose, sodium alginate, hydroxypropyl methyl cellulose, HPMC 1828, HPMC 2906, and HPMC 2910 may be used, and a surfactant, a preservative, a colorant, and a fragrance may be used, if necessary.

The injection according to the present invention may include: a solvent such as distilled water for injection, 0.9% sodium chloride injection, Ringer's injection, dextrose injection, dextrose+sodium chloride injection, PEG, lactated Ringer's injection, ethanol, propylene glycol, non-volatile oil-sesame oil, cottonseed oil, peanut oil, corn oil, ethyl oleate, isopropyl myristate, and benzoic acid benzene; a solubilizing agent such as sodium benzoate, sodium salicylate, sodium acetate, urea, urethane, monoethyl acetamide, butazolidin, propylene glycol, Tweens, nijungtinateamide, hexamine, and dimethylacetamide; a buffer such as a weak acid or a salt thereof (acetic acid and sodium acetate), a weak base and a salt thereof (ammonia and ammonium acetate), an organic compound, a protein, albumin, peptone, and gums; an isotonic agent such as sodium chloride; a stabilizer such as sodium bisulfite (NaHSO₃), carbon dioxide gas, sodium metabisulfite (Na₂S₂O₅), sodium sulfite (Na₂SO₃), nitrogen gas (N₂), and ethylenediaminetetraacetic acid; a sulfating agent such as 0.1% sodium bisulfide, sodium formaldehyde sulfoxylate, thiourea, disodium ethylenediaminetetraacetate, and acetone sodium bisulfite; an analgesic such as benzyl alcohol, chlorobutanol, procaine hydrochloride, glucose, and calcium gluconate; and a suspending agent such as carboxymethyl cellulose sodium, sodium alginate, Tween 80, and aluminum monostearate.

In a suppository according to the present invention, it is possible to use a base such as cacao butter, lanolin, Witepsol, polyethylene glycol, glycerogelatin, methylcellulose, carboxymethyl cellulose, a mixture of stearic acid and oleic acid, Subanal, cottonseed oil, peanut oil, palm oil, cacao butter+cholesterol, lecithin, ranetwax, glycerol monostearate, Tween or Span, Imhausen, monolen (propylene glycol monostearate), glycerin, Adeps solidus, Buytyrum Tego-G, Cebes Pharma 16, hexalide base 95, Cotomar, Hydroxote SP, S-70-XXA, S-70-XX75(S-70-XX95), Hydrokote 25, Hydrokote 711, idropostal, Massa estrarium (A, AS, B, C, D, E, I, T), Massa-MF, Masupol, Masupol-15, Neosupostal-ene, Paramound-B, Suposhiro (OSI, OSIX, A, B, C, D, H, L), suppository base IV types (AB, B, A, BC, BBG, E, BGF, C, D, 299), Supostal (N, Es), Wecobee (W, R, S, M ,Fs), and a tegester triglyceride base (TG-95, MA, 57).

A solid formulation for oral administration includes a tablet, a pill, a powder, a granule, a capsule, and the like, and the solid formulation is prepared by mixing at least one excipient, for example, starch, calcium carbonate, sucrose or lactose, gelatin, and the like with an extract. Further, in addition to a simple excipient, lubricants such as magnesium stearate and talc are also used.

A liquid formulation for oral administration corresponds to a suspension, a liquid for internal use, an emulsion, a syrup, and the like, and the liquid formulation may include, in addition to water and liquid paraffin which are simple commonly used diluents, various excipients, for example, a wetting agent, a sweetener, a fragrance, a preservative, and the like. Examples of a formulation for parenteral administration include an aqueous sterile solution, a non-aqueous solvent, a suspension, an emulsion, a freeze-dried preparation, and a suppository. As the non-aqueous solvent and the suspension, it is possible to use propylene glycol, polyethylene glycol, a vegetable oil such as olive oil, an injectable ester such as ethyl oleate, and the like.

The pharmaceutical composition according to the present invention is administered in a pharmaceutically effective amount. In the present invention, "pharmaceutically effective amount" means an amount sufficient to treat diseases at a reasonable benefit/risk ratio applicable to medical treatment, and an effective dosage level may be determined according to factors including the type of disease of patients, the severity of disease, the activity of drugs, sensitivity to drugs, administration time, administration route, excretion rate, treatment period, and simultaneously used drugs, and other factors well known in the medical field.

The pharmaceutical composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this amount may be easily determined by a person with ordinary skill in the art to which the present invention pertains.

The pharmaceutical composition of the present invention may be administered to a subject in need via various routes. All methods of administration may be expected, but the pharmaceutical composition may be administered by, for example, oral administration, subcutaneous injection, peritoneal administration, intravenous injection, intramuscular injection, paraspinal space (intradural) injection, sublingual administration, buccal administration, intrarectal insertion, intravaginal injection, ocular administration, ear administration, nasal administration, inhalation, spray via the mouth or nose, skin administration, transdermal administration, and the like.

In the present invention, the pharmaceutical composition may be administered by one or more routes selected from the group consisting of oral, subcutaneous, intraperitoneal, intrapulmonary, intranasal, intramuscular, intravenous, intraarterial and topical ocular administration, but is not limited thereto. In the present invention, the topical ocular administration may be one selected from the group consisting of intraconjunctival administration, intravitreal administration, subretinal administration, suprachoroidal administration, subconjunctival administration, and administration under the Tenon's capsule, but is not limited thereto.

The pharmaceutical composition of the present invention is determined by the type of drug that is an active ingredient, as well as various related factors such as the disease to be treated, the route of administration, the age, sex, and body weight of a patient, and the severity of the disease.

As used herein, the "subject" refers to a subject in need of treatment of a target disease, and more specifically, may be a mammal such as a human or a nonhuman primate, a mouse, a rat, a dog, a cat, a horse, and a cow, but is not limited thereto. The subject may be a subject in need of treatment of a retinal or choroidal disease, but is not limited thereto.

The "administration" as used herein refers to the provision of a predetermined composition of the present invention to a subject in need thereof by any suitable method.

As used herein, the "prevention" refers to all actions that suppress or delay the onset of a target disease, and the "treatment" refers to all actions that ameliorate or beneficially change a target disease and the resulting metabolic abnormalities by administration of the pharmaceutical composition according to the present invention, and the "amelioration" refers to all actions that reduce a target disease and associated parameters, for example, the severity of symptoms, by administration of the composition according to the present invention.

Further, the present invention provides a quasi-drug composition for the prevention or treatment of a retinal or choroidal disease, the quasi-drug composition comprising a plasmalemma vesicle-associated protein (PLVAP) protein, an expression or activity activator of the protein, or a nucleotide sequence encoding the PLVAP protein, as an active ingredient.

The quasi-drug composition refers to an article which has a milder action than a drug among articles used for the purpose of diagnosing, treating, ameliorating, alleviating, treating, or preventing a human or animal disease, and for example, according to the Pharmaceutical Affairs Act, the quasi-drug is an article that is not used for pharmaceutical purposes, and includes fiber and rubber products used for the treatment and prevention of human and animal diseases, those that have little or no direct effect on the human body and are similar to those that are not instruments or machines, and bactericidal and pesticides to prevent infectious diseases, and the like.

In the present invention, the quasi-drug composition may be used by being formulated into a dosage form of an ophthalmic composition, and may be used, for example, as one or more formulations selected from the group consisting of an ophthalmic solution, a collyrium, an ophthalmic ointment, an injection, and an eyewash, but is not limited thereto.

Since the present invention may be modified into various forms and include various exemplary embodiments, specific exemplary embodiments will be illustrated in the drawings and described in detail in the Detailed Description. However, the description is not intended to limit the present invention to the specific exemplary embodiments, and it is to be understood that all the changes, equivalents, and substitutions belonging to the spirit and technical scope of the present invention are included in the present invention. When it is determined that the detailed description of the related publicly known art in describing the present invention may obscure the gist of the present invention, the detailed description thereof will be omitted.

### [Modes of the Invention]

Hereinafter, preferred examples for helping with understanding of the present invention will be suggested. However, the following examples are provided only so that the present invention may be more easily understood, and the content of the present invention is not limited by the following examples.

### [Examples]

### Example 1. Construction of optimal plasmid DNA expressing PLVAP

### 1-1. Construction of optimal plasmid DNA expressing PLVAP

PLVAP plasmid DNA was constructed using promoters, which are specifically expressed in cytomegaloviruses (CMV) or vascular endothelial cells, as non-specific cell-expressed promoters. The promoters specifically expressed in the vascular endothelial cells were as follows: CD144 (VE-cadherin), TIE1, FLT1 (VEGFR1), or EMCN (Endomucin). eGFP was inserted as a reporter gene upstream of a PLVAP sequence in order to confirm successful plasmid insertion and gene expression. In this case, integrating linkers were used depending on the size of the plasmid and are as follows: attB 1/2, an internal ribosome entry site (IRES), and a Thosea asigna virus 2A peptide (T2A). To overexpress the PLVAP, promotereGFP-PLVAP plasmid DNA was constructed using the following method. The backbone of an underlying plasmid was constructed using a Design Vector Studio tool commercially available from VectorBuilder, and a base sequence of a CMV, CD144, TIE1, FLT1, or EMCN promoter was inserted thereinto. Thereafter, a base sequence of eGFP was inserted into the open reading frame 1 (ORF1), a base sequence of PLVAP was inserted into the open reading frame 2 (ORF2), and attB 1/2, IRES, or T2A, which is a base sequence of an integrating linker (serving to continuously express and automatically cleave two polypeptides) allowing the expression of both the eGFP and the PLVAP, was inserted between ORF 1 and ORF2. An SV40 late mRNA polyadenylation signal sequence was inserted at the end of ORF2, and a CMV promoter and an antibiotic-resistance gene were inserted. Then, a vector was customized using the corresponding plasmid map. A structure of the constructed vector is shown in FIG. 1.

**[Table 1]**

| | **Sequence (5'→3')** | **SEQ ID NO:** |
|---|---|---|
| hPLVAP | CCCGGCAGACCCAGCTAGCGCTGGAGGAGAAGGCGGTG CTGCGGAAGGAACGAGACAACCTGGCCAAGGAGCTGGA AGAGAAGAAGAGGGAGGCGGAGCAGCTCAGGATGGAG CTGGCCATCAGAAACTCAGCCCTGGACACCTGCATCAA GACCAAGTCGCAGCCGATGATGCCAGTGTCAAGGCCCA TGGGCCCTGTCCCCAACCCCCAGCCCATCGACCCAGCTA GCCTGGAGGAGTTCAAGAGGAAGATCCTGGAGTCCCAG AGGCCCCCTGCAGGCATCCCTGTAGCCCCATCCAGTGGC TGA | 1 |
| mPLVAP | CGCAGCTGGCTCTGGAGGAGAAGGCGGCGCTGCGCGCG CAGCGGGACAACCTAGAGCGCGAGCTGGAGGCGCGTAA GCGGGAGCTGGAGCAACTGCGCACCGAGGTGGATGTGC GCATTAGCGCGCTGGACACCTGCGTCAAGGCCAAGTCG CTGCCAGCCGTCCCGCCGAGAGTCTCAGGCCCACCCCCG AACCCTCCACCCATTGATCCAGCTAGCCTGGAGGAATTC AAGAAAAGGATCCTGGAGTCTCAGCGGCTCCCTGTAGT CAACCCTGCTGCCCAACCCAGCGGTTGA | 2 |
| h/mCMV | GACATTGATTATTGACTAGTTATTAATAGTAATCAATTA CGGGGTCATTAGTTCATAGCCCATATATGGAGTTCCGCG TTACATAACTTACGGTAAATGGCCCGCCTGGCTGACCGC CCAACGACCCCCGCCCATTGACGTCAATAATGACGTATG TTCCCATAGTAACGCCAATAGGGACTTTCCATTGACGTC AATGGGTGGAGTATTTACGGTAAACTGCCCACTTGGCAG TACATCAAGTGTATCATATGCCAAGTACGCCCCCTATTG ACGTCAATGACGGTAAATGGCCCGCCTGGCATTATGCCC AGTACATGACCTTATGGGACTTTCCTACTTGGCAGTACA TCTACGTATTAGTCATCGCTATTACCATGGTGATGCGGT TTTGGCAGTACATCAATGGGCGTGGATAGCGGTTTGACT CACGGGGATTTCCAAGTCTCCACCCCATTGACGTCAATG GGAGTTTGTTTTGGCACCAAAATCAACGGGACTTTCCAA AATGTCGTAACAACTCCGCCCCATTGACGCAAATGGGC GGTAGGCGTGTACGGTGGGAGGTCTATATAAGCAGAGC T | 3 |
| hCD144 | TGATGCTGCAGCCAAGGGTCCCCACAGCCAGGCTCTTTG CAGGTGCTGGGTTCAGAGTCCCAGAGCTGAGGCCGGGA GTAGGGGTTCAAGTGGGGTGCCCCAGGCAGGGTCCAGT GCCAGCCCTCTGTGGAGACAGCCATCCGGGGCCGAGGC AGCCGCCCACCGCAGGGCCTGCCTATCTGCAGCCAGCCC AGCCCTCACAAAGGAACAATAACAGGAAACCATCCCAG GGGGAAGTGGGCCAGGGCCAGCTGGAAAACCTGAAGG GGAGGCAGCCAGGCCTCCCTCGCCAGCGGGGTGTGGCT CCCCTCCAAAGACGGTCGGCTGACAGGCTCCACAGAGC TCCACTCACGCTCAGCCCTGGACGGACAGGCAGTCCAA CGGAACAGAAACATCCCTCAGCCCACAGGCACGGTGAG TGGGGGCTCCCACACTCCCCTCCACCCCAAACCCGCCAC CCTGCGCCCAAGATGGGAGGGTCCTCAGCTTCCCCATCT GTAGAATGGGCATCGTCCCACTCCCATGACAGAGAGGC TCC | 4 |
| mCD144 | GGCTCTGTGGAGAAGCTGAGGTAGCTGGGTTTCTGTCTG GGTTACTCTGTCCAGCGAGGAAACAAGTACCTTAGACCC ACTAAGCCTCTGCTTTCTGAACTGTAAAGTGGGGGATAT GACACCTGCCTCCCAGGGATGGCTGAATGCTCTGGCAG AAGCTTAGAGCCCCCACAGCTACCCCTAGGCTCACAGCT CCTCCGATGAGACCTAGAATTGAGGTATGAGTTGAATAC CCCAGGCAGGTCCAAGGCTTCCACGGGCCCAGGCTGAC CAAGCTGAGGCCGCCCACCGTAGGGCTTGCCTATCTGCA GGCAGCTCACAAAGGAACAATAACAGGAAACCATCCCG AGGGGAAGTGGGCCAGGGCCAGTTGGAAAACCTGCCTC CCTCCCAGCCTGGGTGTGGCTCCCCTCTCCCCTCCTGAG GCAATCAACTGTGCTCTCCACAAAGCTCGGCCCTGGACA GACT | 5 |
| hTIE1 | GGGGTGCAGGAGACCCACTGTGGATAGACGTGGACAGG CCTGAGGAGACAAGCACCATCATCCGTGGCCTCAACGC CAGCACGCGCTACCTCTTCCGCATGCGGGCCAGCATTCA GGGGCTCGGGGACTGGAGCAACACAGTAGAAGAGTCCA CCCTGGGCAACGGGCTGCAGGCTGAGGGCCCAGTCCAA GAGAGCCGGGCAGCTGAAGAGGGCCTGGATCAGCAGCT GATCCTGGCGGTGGTGGGCTCCGTGTCTGCCACCTGCCT CACCATCCTGGCTGCCCTTTTAACCCTGGTGTGCATCCG CAGAAGCTGCCTGCATCGGAGACGCACCTTCACCTACCA | 6 |
| | GTCAGGCTCGGGCGAGGAGACCATCCTGCAGTTCAGCT CAGGGACCTTGACACTTACCCGGCGGCCAAAACTGCAG CCCGAGCCCCTGAGCTACCCAGTGCTAGAGTGGGAGGA CATCACCTTTGAGGACCTCATCGGG | |
| mTIE1 | GTCTGTGTCTCTCTGGCCCTAACCGATCTTCAATCCCAG CCA | 7 |
| hFLT1 | TCTTCAGAGGCGCCTTCACTTTCATCACTCTGTTCATGCT GCTCGTCTGCCTGCAAGACCTTTTCCCTCCCTCTGTGTCT CCTCCTCTCCTGCTGGCAAGATCCAGCCCAGTAATCACC ATCTCTGGGAGCTCCTCCTTGTAGCTCCCAGACAGCACT TGCCATTTCTTCTGCTCTCCTGTAGCCCTGAGGTGTACTC CTCCTTCAGTAATTC | 8 |
| mFLT1 | GGGGGACCCCTTGACGTCACTGGAAGGAGGTGCCGGGG TAGGAAGTGGGCTGGGGAAAGGTTATAAATCGCCCCCG CCCTCGGCTGCACTTCAGCGAGGTCCTTGAGAGGCTCGG AGCGCGGTGGCGGACACTCCCGGGAGGTAGTGCTAGTG GTGGTGGCTGCTGCTCGGAGCGGGCTCCGGGACTCAAG CGCAGCGGCTAGCGGACGCGGGACGGCGAGGATCCCCC CACACCACCCCCCTCGGCTGCAGGCGCGGAGAAGGGCT CTCGCGGCGCCAAGCAGAAGCAGGAGGGGACCGGCTCG AGCGGCTGCGCCGTCGGCCTCGGAGAGCGCGGGC | 9 |
| hEMCN | ACATTCACTACACCTTTTCCATTTGCTAATAAGGCCCTG CCAGGCTGGGAGGGAATTGTCCCTGCCTGCTTCTGGAGA AAGAAGATATTGACACCATCTACGGGCACCATGGAACT GCTTCAAGTGACCATTCTTTTTCTTCTGCCCAGTATTTGC AGCAGTAACAGCACAGGTATTTTTGTCTCTTATTCCTCT GGTGGTACGGATTGGAAATCACTAGTAGGTGACTCAGC CTCAGTTTACTTTCAGGGATCTTCCCCTGACACTTAGAT GCAGCAATTCCTTTGATGAGTTGAGTGTTTGGATGTTGA CTTGGAGTGCGTGGAGAAGGCTTGAGAGAGACGGTATT TGTTTCTACTGAAGGGAAATGTTTGCGGGTACAGCAGCC TGAAAGCATGTTCGATGAAATCACAAAGTACTGGA | 10 |
| mEMCN | AGGGGAATGCCAGGGCCAAGAAGTGGGAGGGGGAGGG GGAGAGGAGTGGGGGGGTATGGGGGACTTTTGGAATAG CAGTGGAAATGTAAATGAAGAAAATATCTAATAAAAAT ATTTAAAAAGAAAGAAAGAAATGTCTTGAAAACCTCTC | 11 |
| | TGGGCAGTAATAAGAGGCCAGCTTACTTCCTCAAGAGC AGAACGTGATCCAGTTCATACTTGTATTTGCCTCTAGAA GGTGGTCTTCACTGACTTGGCTTATGAGTTGAGAAGAGA GAAGAAATGCAATTCCTTGTGGATTTTGTACTCAAATCC AGACCTTGGGAAGTCACAGCCTTTTCAGCCGACCTGGGG GGCTACTTGGTGAGGTCTCTTTACAGCTTCTCTAAAATG CACTTTCAAAACAAAAGTTAGAAAGCTCTCTGGCCCTTT CTCAAGAAGGGTCTCTTCCTGTTCAAATCCCATCCTGAC CTTTTTGTTGAAGAAGAGTAAAATAATGCAGTAACAAA AACACAAGTGGCTGCCAAAGGAAGAGAGAGGAAAAAA AAAGAAGTTCCTGTAAGGGGCTGAAACAATAAATCTCC TCTCTGCTGACCTCCCAAAGGGAGTGCGTGTATTTCCTC TAGTTCTTTATCAGAATCCCCAAAATAAGTAGGAATGGG CAGCGTCTGCTCACAGTCAGTACACCTTTCCCATTTGCT AATACGGCCTGGCCAGGCTGAGAGGAATCCTTCCCTGCC TTACCCT | |
| attB1 | ACAAGTTTGTACAAAAAAGCAGGCT | 12 |
| attB2 | ACCACTTTGTACAAGAAAGCTCGGT | 13 |
| IRES | GCCCCTCTCCCTCCCCCCCCCCTAACGTTACTGGCCGAA GCCGCTTGGAATAAGGCCGGTGTGCGTTTGTCTATATGT TATTTTCCACCATATTGCCGTCTTTTGGCAATGTGAGGG CCCGGAAACCTGGCCCTGTCTTCTTGACGAGCATTCCTA GGGGTCTTTCCCCTCTCGCCAAAGGAATGCAAGGTCTGT TGAATGTCGTGAAGGAAGCAGTTCCTCTGGAAGCTTCTT GAAGACAAACAACGTCTGTAGCGACCCTTTGCAGGCAG CGGAACCCCCCACCTGGCGACAGGTGCCTCTGCGGCCA AAAGCCACGTGTATAAGATACACCTGCAAAGGCGGCAC AACCCCAGTGCCACGTTGTGAGTTGGATAGTTGTGGAAA GAGTCAAATGGCTCTCCTCAAGCGTATTCAACAAGGGG CTGAAGGATGCCCAGAAGGTACCCCATTGTATGGGATCT GATCTGGGGCCTCGGTGCACATGCTTTACATGTGTTTAG TCGAGGTTAAAAAAACGTCTAGGCCCCCCGAACCACGG GGACGTGGTTTTCCTTTGAAAAACACGATGATAATATGG CCACAACC | 14 |
| T2A | GGAAGCGGAGAGGGCAGGGGAAGTCTTCTAACATGCGG GGACGTGGAGGAAAATCCCGGCCCC | 15 |
| eGFP | ATGGTGAGCAAGGGCGAGGAGCTGTTCACCGGGGTGGT GCCCATCCTGGTCGAGCTGGACGGCGACGTAAACGGCC ACAAGTTCAGCGTGTCCGGCGAGGGCGAGGGCGATGCC ACCTACGGCAAGCTGACCCTGAAGTTCATCTGCACCACC GGCAAGCTGCCCGTGCCCTGGCCCACCCTCGTGACCACC CTGACCTACGGCGTGCAGTGCTTCAGCCGCTACCCCGAC CACATGAAGCAGCACGACTTCTTCAAGTCCGCCATGCCC GAAGGCTACGTCCAGGAGCGCACCATCTTCTTCAAGGA CGACGGCAACTACAAGACCCGCGCCGAGGTGAAGTTCG AGGGCGACACCCTGGTGAACCGCATCGAGCTGAAGGGC ATCGACTTCAAGGAGGACGGCAACATCCTGGGGCACAA GCTGGAGTACAACTACAACAGCCACAACGTCTATATCAT GGCCGACAAGCAGAAGAACGGCATCAAGGTGAACTTCA AGATCCGCCACAACATCGAGGACGGCAGCGTGCAGCTC GCCGACCACTACCAGCAGAACACCCCCATCGGCGACGG CCCCGTGCTGCTGCCCGACAACCACTACCTGAGCACCCA GTCCAAGCTGAGCAAAGACCCCAACGAGAAGCGCGATC ACATGGTCCTGCTGGAGTTCGTGACCGCCGCCGGGATCA CTCTCGGCATGGACGAGCTGTACAAG | 16 |

### 1-2. Confirmation of expression of plasmid DNA in human choroidal vascular endothelial cells

Primarily cultured human choroidal vascular endothelial cells collected from eyeballs donated for corneal transplantation under the consent of the Human-Derived Material Research ICF were treated with the plasmid DNA to check whether the plasmid DNA constructed in Example 1-1 was well expressed in the vascular endothelial cells. Lipofectamine^{™} 3000, which is an in-vitro transfection reagent commercially available from Invitrogen was added to the plasmid DNA so that a final concentration of the plasmid DNA became 2 µg/mL. The human choroidal vascular endothelial cells were treated with the plasmid. After 2 days, the culture medium was changed, and the cells were cultured for another 2 days to check whether the PLVAP plasmid was expressed.

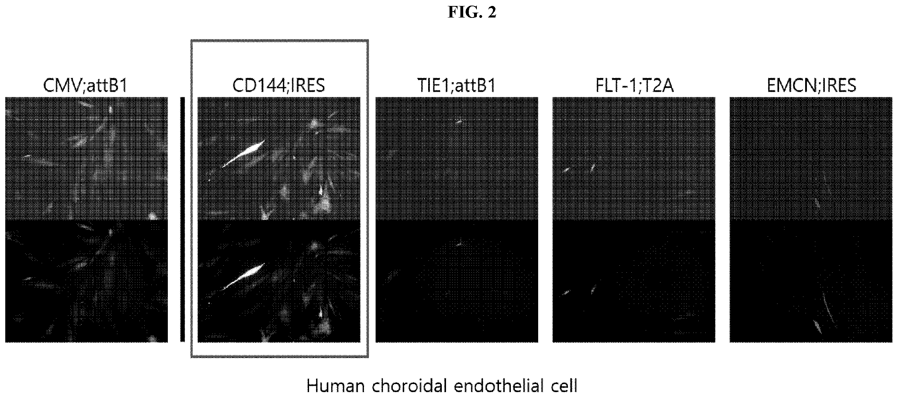

As shown in FIG. 2, all the plasmid DNAs were effectively introduced into the human choroidal vascular endothelial cells, and it was confirmed that the PLVAP was expressed in the vascular endothelial cells after transfection. Among these, the plasmid DNA containing CD144 as the promoter had the highest expression level.

### 1-3. Confirmation of expression of plasmid DNA in mouse retina and choroid

The plasmid DNA was subretinally injected into wild-type C57BL/6 mice, and RT-PCR was performed to check whether the PLVAP gene in the plasmid DNA including CD144 and IRES constructed in Example 1-1 was well expressed in the mouse retina and choroid. GAPDH was used as a housekeeping gene. Total RNA was extracted using an RNeasy Plus Mini kit (Qiagen, Hilden, Germany) according to the manufacturer's instructions, and reversely transcribed into cDNA using a PrimeScript^{™} 1st strand cDNA Synthesis kit (Takara, Shiga, Japan). Quantitative real-time PCR (RT-PCR) was performed using a real-time PCR detection system (Bio-Rad TM CFX96; Bio-Rad Laboratories, Inc., Hercules, CA, USA) using SsoAdvanced Universal SYBR Green Supermix (Bio-Rad, CA, US) together with primers listed in Table 2. An RT-PCR data was analyzed using amplification software (Bio-Rad CFX Manager; Bio-Rad Laboratories, Inc.).

**[Table 2]**

| RT-PCR primer | | | |
|---|---|---|---|
| | | **Sequence (5'→3')** | SEQ ID NO: |
| Human | GAPDH | F: CCTCCAAGGAGTAAGACCCC | 17 |
| | | R: AGGGGTCTACATGGCAACTG | 18 |
| | PLVAP | F:AGAACTCAGACCTCCAACGC | 19 |
| | | R: TCTCCACCTTCTGTTTCGCC | 20 |
| Mouse | GAPDH | F: TGTCCGTCGTGGATCTGAC | 21 |
| | | R: CCTGCTTCACCACCTTCTTG | 22 |
| | PLVAP | F: GCTGGTACTACCTGCGCTATT | 23 |
| | | R: CCTGTGAGGCAGATAGTCCA | 24 |

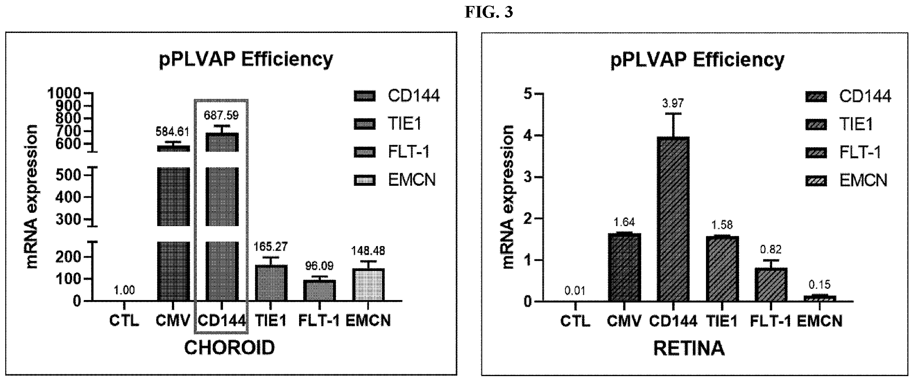

As shown in FIG. 3, all the plasmid DNAs significantly induced PLVAP expression. Among them, the plasmid DNAs comprising the CMV and CD144 promoters had significantly high expression levels.

### Example 2. Confirmation of therapeutic effect on age-related macular degeneration through injection of plasmid DNA expressing PLVAP

### 2-1. Construction of age-related macular degeneration mouse model

An age-related macular degeneration model was constructed by mating a PLVAP flox mouse with a VE-cadherin-cre^{ERT2} mouse to specifically deplete PLVAP in mouse vascular endothelial cells. Specifically, VE-cadherin-cre^{ERT2}; PLVAP flox (VEcad-Cre^{ERT2}; PLVAP^{flox/flox}; hereinafter referred to as PLVAP^{iECKO}) genotype mice, which were inducible endothelial cell-specific knockout mice combined by mating a VE-cadherin-cre^{ERT2} mouse corresponding to mouse strain 1 with a PLVAP flox mouse corresponding to mouse strain 2 were used. A cyclic recombinase (Cre) activated by a tamoxifen-inducible estrogen receptor (ERT2) was made when a tamoxifen drug was introduced into PLVAP^{iECKO}, FLOX cassettes positioned upstream and downstream of a PLVAP gene exon were ligated into a cyclic structure, and a portion of a PLVAP activator gene present between the cassettes was removed to make use of a system serving to prevent the PLVAP gene from being normally transcribed.

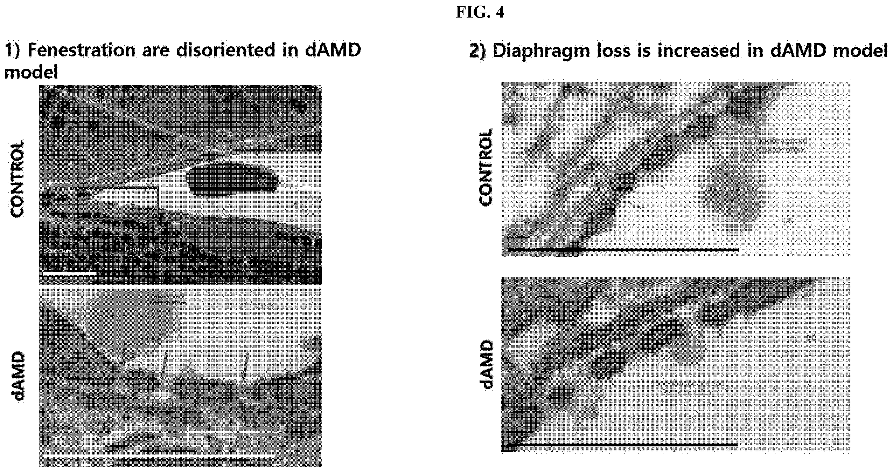

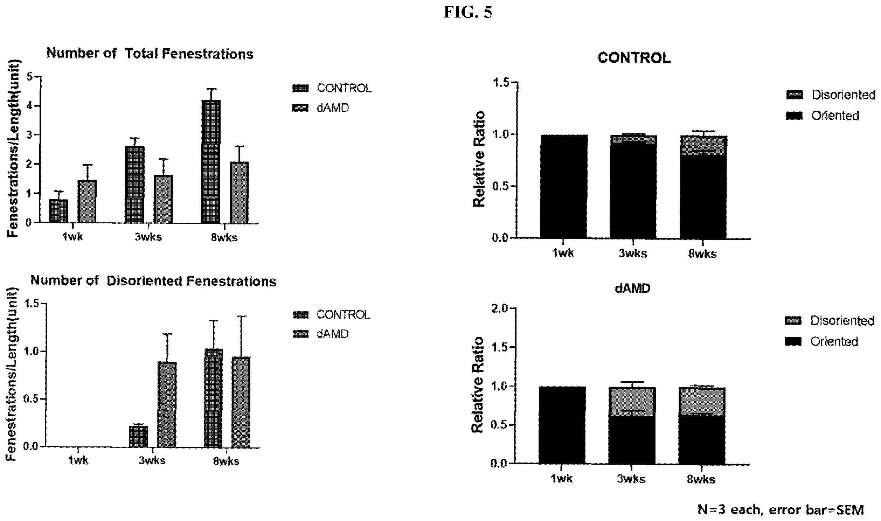

As show in FIGS. 4 and 5, a decrease in the total number of fenestrations in a choriocapillary vessel and a change in directionality of the fenestrations were caused, and the diaphragms of the fenestration disappeared in the AMD model of the present invention. These results were consistent with the histological findings observed in human macular degeneration.

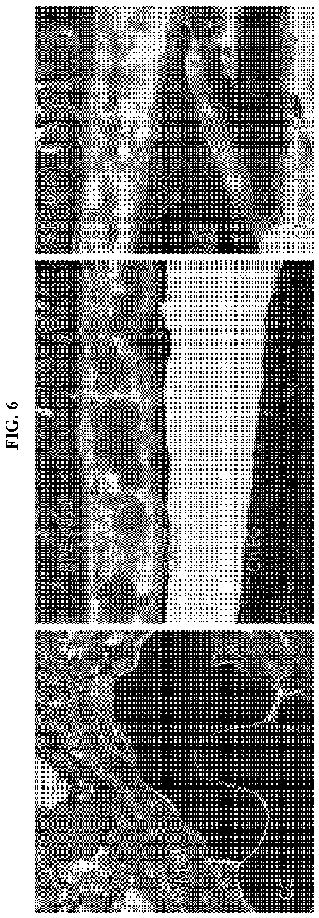

Also, as shown in FIG. 6, metabolic waste in the retina was accumulated in a subretinal space and the Bruch's membrane because the metabolic wastes did not normally pass through the choroid vessel due to a change in the choriocapillary vessel, which had a pattern similar to lipid droplets or drusen found in patients with dry macular degeneration. Therefore, it was confirmed that it was suitable to use the genetically modified mice as the age-related macular degeneration animal model.

### 2-2. Confirmation of improvement of choriocapillary vessel structure by administration of plasmid DNA in age-related macular degeneration mouse model

It was confirmed whether the structure of the choriocapillary vessel was restored when the PLVAP plasmid DNA was administered into the subretinal space in the AMD model shown in Example 2-1. The plasmid DNA was delivered in vivo using an in vivo-jetPEI product commercially available from Polyplus Transfection. A reagent obtained by mixing 4 µg of the plasmid DNA including CMV and CD 144 promoters of Example 1-1 and 0.4 µL of an in-vivo transfection reagent in 10 µL of a 5% glucose solution was injected once into the subretinal space of a PLVAP^{ECKO} mouse model at a volume of 4 µL using a 38G microneedle (Incyto Co., Ltd.). As the control, a vehicle, which was a mixture of a plasmid-free 5% glucose solution and an in-vivo transfection reagent, was injected once into the subretinal space.

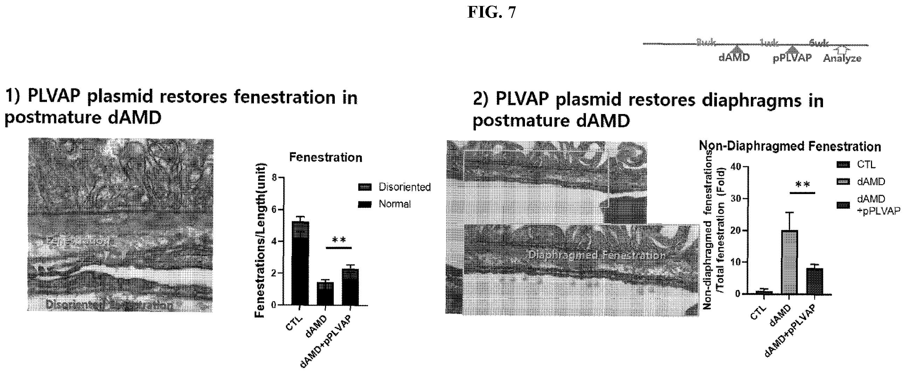

As shown in FIG. 7, it was confirmed that the choriocapillary vessel was restored into a normal choriocapillary vessel as the number of fenestrations which had decreased was recovered, and the membranes constituting the fenestrations were restored in the constructed animal model.

The results show that the PLVAP plasmid DNA of the present invention normalized the choriocapillary vessel structure in the age-related macular degeneration mice.

### 2-3. Confirmation of mechanism of prevention and treatment of age-related macular degeneration by administration of plasmid DNA

The PLVAP plasmid was administered according to the method of Example 2-2. It was confirmed whether lipid droplets and drusen, which are symptoms of dry macular degeneration patients, were ameliorated when the plasmid PLVAP was administered into the AMD model, and whether the metabolic waste in the retina was well discharged.

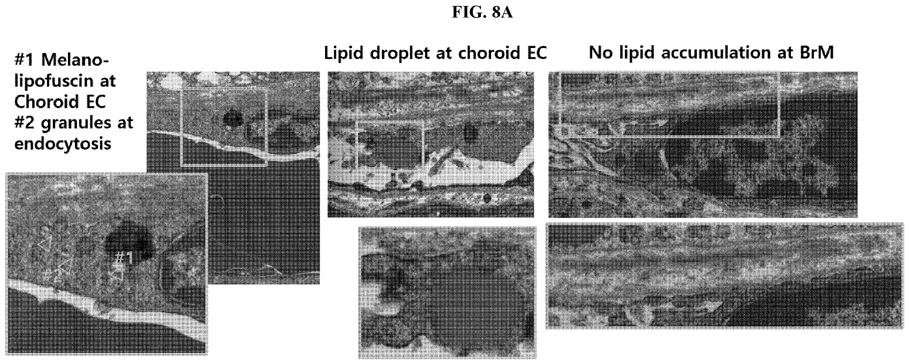

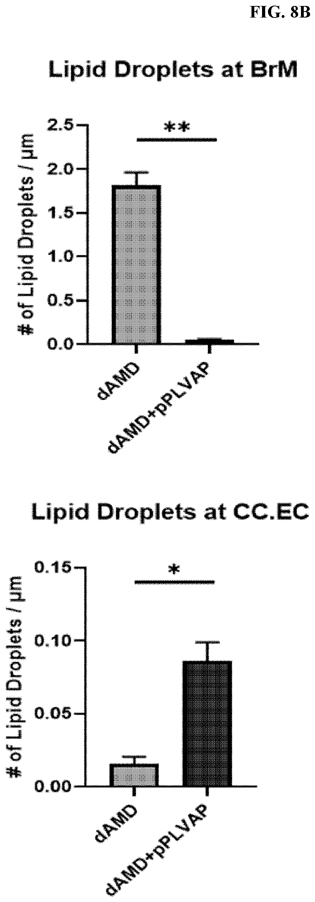

As shown in FIGS. 8A and 8B, all the lipid droplets which had been accumulated in the Bruch's membrane in the AMD model disappeared. Based on the results, it was confirmed that a change in the choriocapillary vessel structure induced by the PLVAP plasmid DNA promoted the excretion of lipid metabolites from the retina. Specifically, it was confirmed that melano-lipofuscin, granules and lipid droplets, which are representative lipid metabolites of the retinal pigment epithelium (RPE), were incorporated into endothelial cells (EC) from the choriocapillary vessel and Bruch's membrane.

Therefore, by administering the PLVAP plasmid DNA, it is possible to prevent and treat the age-related macular degeneration by preventing a basal deposit between the drusen and the Bruch's membrane in age-related macular degeneration.

### 2-4. Confirmation of inhibitory effect of administration of plasmid DNA on onset and early progression of age-related macular degeneration

An inhibitory effect of administration of the PLVAP plasmid DNA on the onset and early progression of age-related macular degeneration was evaluated. The PLVAP plasmid was administered according to the method of Example 2-2. At 8 weeks of age in mice, macular degeneration was induced, and simultaneously, PLVAP plasmid DNA comprising CMV and CD144 promoters was injected into the eyes of the age-related macular degeneration model, followed by an incubation time of 2 weeks to allow enough time for the PLVAP plasmid DNA to be fully expressed. The progression of retinal degeneration was analyzed by ultra-widefield fundus photography.

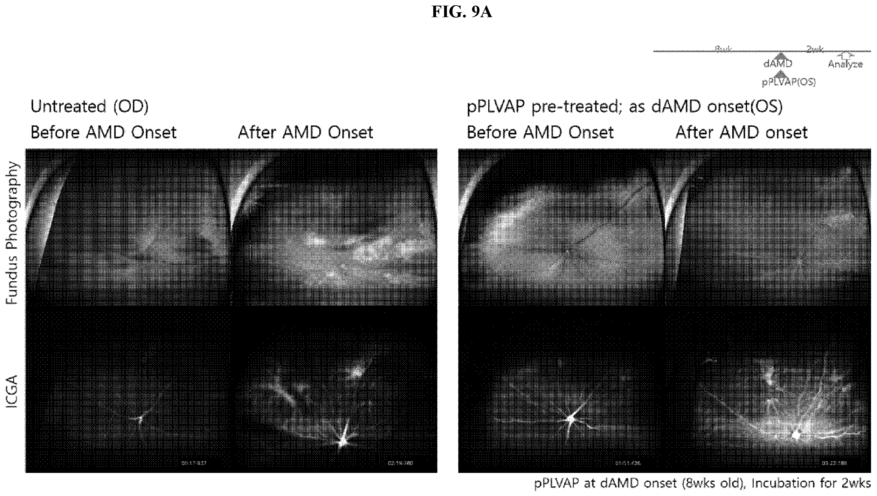

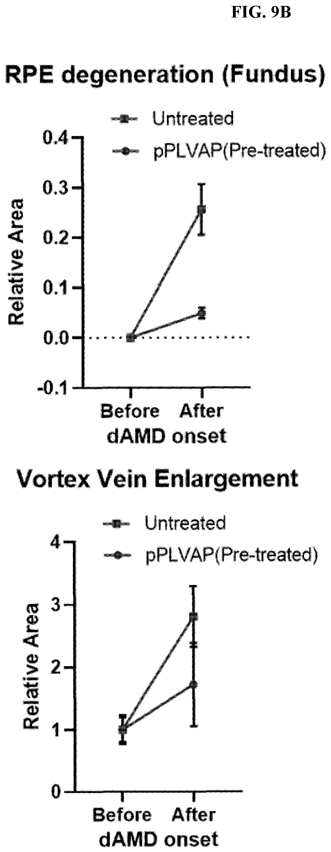

As shown in FIGS. 9A and 9B, the progression of retinal degeneration was suppressed in the eyes administered the PLVAP plasmid compared to the control. Specifically, it was confirmed that the degeneration of the retinal pigment epithelium (RPE) was significantly suppressed and the expansion of the vortex vein was significantly reduced.

This suggests that there is an effect of preventing and suppressing the onset and progression of the disease using PLVAP plasmid in the early stage of age-related macular degeneration.

### 2-5. Confirmation of therapeutic effect of administration of plasmid DNA on age-related macular degeneration

Meanwhile, in order to verify the therapeutic effect, age-related macular degeneration was induced. After 2 weeks, the symptoms of macular degeneration were sufficiently expressed, and the PLVAP plasmid was then administered thereinto. The expression time was set at 2 weeks after injection. The PLVAP plasmid was administered according to the method of Example 2-2.

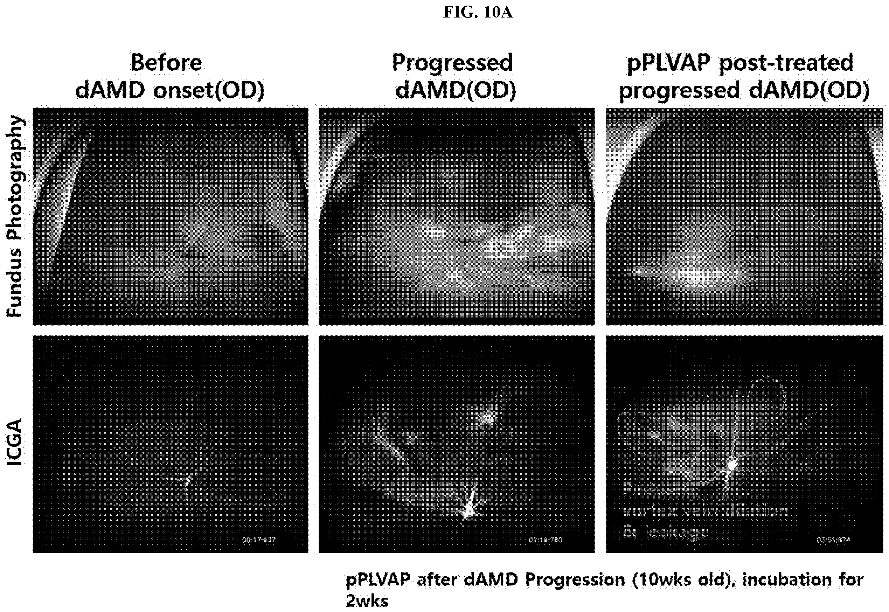

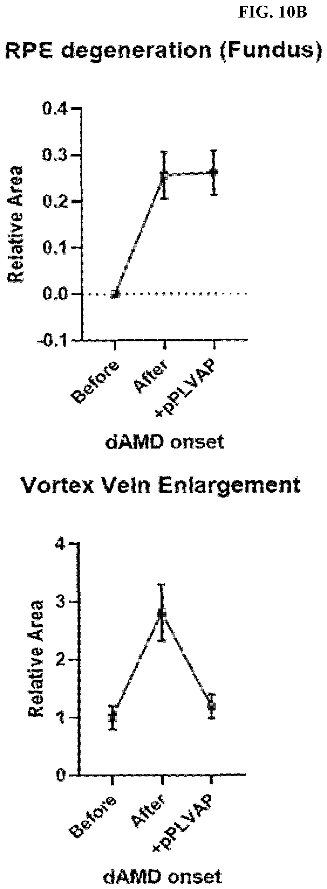

As shown in FIGS. 10A and 10B, the progression of retinal degeneration was inhibited when the PLVAP plasmid was administered. Also, the results of choroid angiography (indocyanine green angiography, ICGA) show that the expansion of a vortex vein was reduced, and the leakage from the choroid vessel was reduced. From these results, it can be expected that the PLVAP plasmid had a therapeutic effect on both dry and wet age-related macular degeneration.

### Example 3. Confirmation of effect of PLVAP plasmid DNA on restoration of impaired visual function

To evaluate the visual function of the dry age-related macular degeneration model, electroretinogram (ERG) was performed. The electroretinogram (ERG) plays an important role in diagnosing and studying abnormalities in retinal function. A decrease in amplitudes of a and b waves was observed under dark adaptation. Here, a decrease in the amplitude of the a wave indicates a malfunction of photoreceptors, and a decrease in the amplitude of the b wave indicates a malfunction of bipolar cells. An electroretinogram method was performed as described in [Documenta Ophthalmologica volume 130, pages 1-12 (2015)]. The electroretinogram was performed 4 weeks after injecting the PLVAP plasmid DNA into the eyeball of the age-related macular degeneration model.

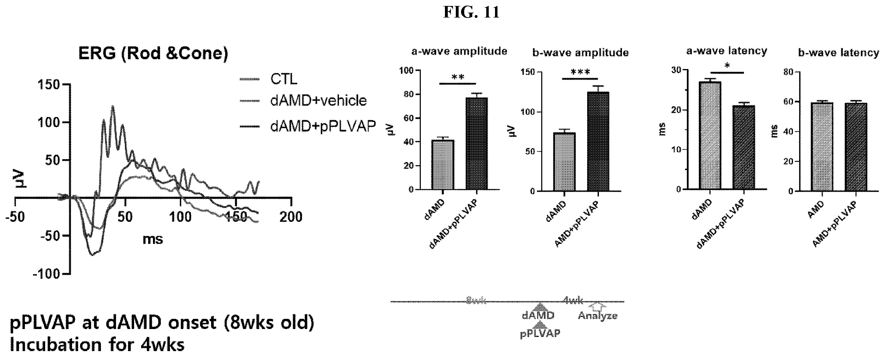

As shown in FIG. 11, it was confirmed that the amplitudes of a and b waves increased and the latency decreased to restore the function of visual cells in the experimental group into which the PLVAP plasmid was subretinally administered in the age-related macular degeneration model, compared to the control. This indicates that the PLVAP plasmid DNA of the present invention is effective in restoring the impaired visual function caused by age-related macular degeneration.

### Example 4. Confirmation of effect of PLVAP plasmid DNA on wet (exudative) age-related macular degeneration

From the results of Examples, it was confirmed that the PLVAP plasmid DNA had a normalizing effect on the choroid vessel. Based on the results, an antiangiogenic effect on wet age-related macular degeneration was checked using a laser-induced choroidal neovascularization model. At 7 weeks of age, 4 µL of a PLVAP plasmid solution was injected into the eyevalls of the model in the same manner as in the preparation and injection of the PLVAP plasmid solution used in Example 2-2. After one week, a choroidal neovascularization model mimicking wet macular degeneration was constructed by applying a laser to the 8-week-old mice. After laser irradiation, 2 weeks were set as a time for neovascularization to sufficiently proceed. Thereafter, the mouse eyeballs were collected to perform a histological examination.

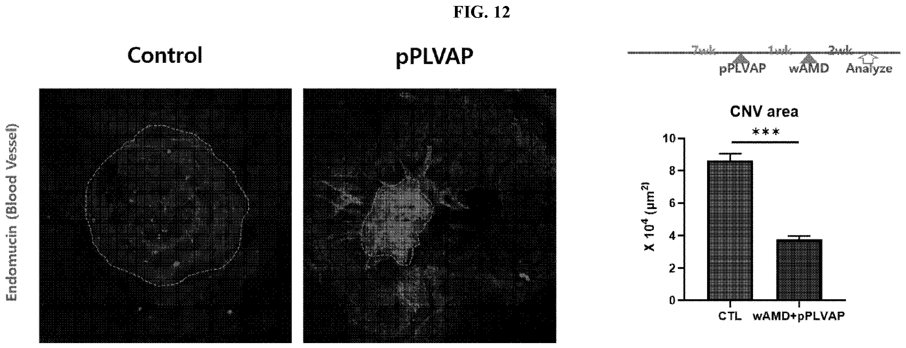

As shown in FIG. 12, it was confirmed that, when the PLVAP plasmid DNA was administered, the PLVAP plasmid DNA inhibited choroidal neovascularization compared to the control, indicating that the PLVAP plasmid was effective in treating wet age-related macular degeneration.

### Example 5. Confirmation of therapeutic effect of injection of PLVAP-expressing plasmid DNA on diabetic retinopathy/chorioretinopathy

### 5-1. Construction of diabetic chorioretinopathy mouse model

Diabetic chorioretinopathy is a disease that causes retinal degeneration due to structural and functional changes in choroid vessels by prolonged diabetes to cause vision loss. It was reported that various changes in choroid vessels such as expansion of a choroid vessel, loss of a choriocapillary vessel, and the like were observed in diabetic chorioretinopathy. Among these, a change in a choriocapillary vessel was most important for maintaining the function of the retina. To construct a diabetic chorioretinopathy animal model, first, streptozotocin (STZ; Sigma) was intraperitoneally administered into 7- to 8-week-old male C57BL6/J mice at a dose of 200 mg/kg. After one week, blood glucose was measured to check whether hyperglycemia was induced . The mice whose blood sugar was induced to 400 mg/dL or more were selected and used for subsequent experiments. Approximately 8 weeks after STZ administration, early symptoms of diabetic chorioretinopathy began to appear.

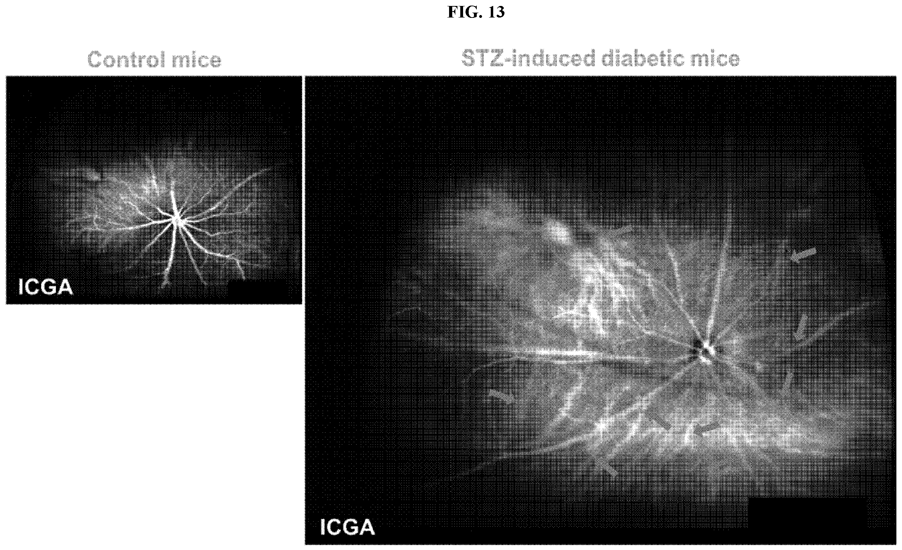

As shown in FIG. 13, the laboratory animals in which diabetes was induced showed a phenomenon in which a choroid vessel expanded in a similar manner as in the age-related macular degeneration animal model.

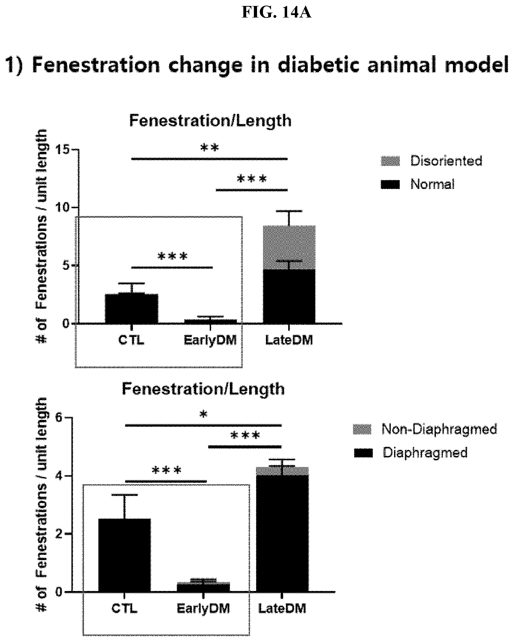

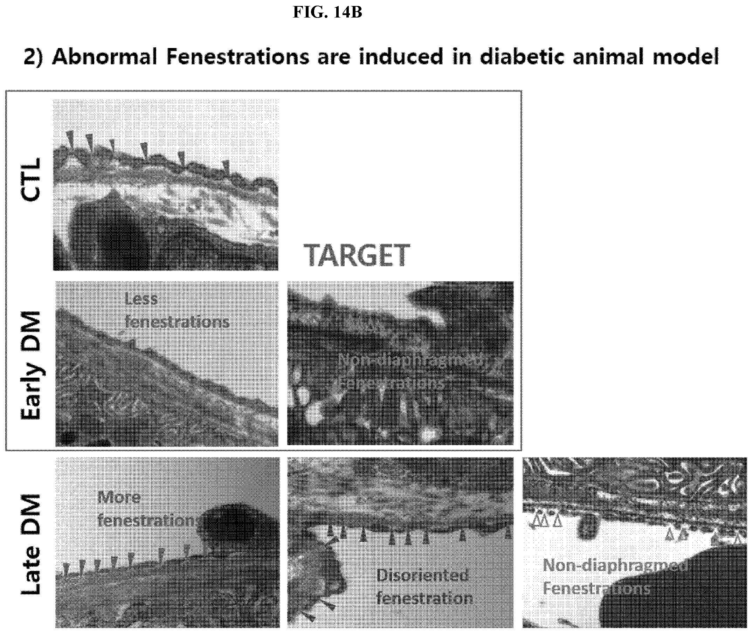

Also, as shown in FIGS. 14A and 14B, the characteristics of diabetic chorioretinopathy, such as a decrease in the total number of fenestrations in a choriocapillary vessel and an increase in the ratio of fenestrations whose diaphragm was lost, were observed. As a result, it was confirmed that the animal model was suitable for use as a diabetic chorioretinopathy animal model.

### 5-2. Confirmation of improvement of choriocapillary vessel structure in diabetic chorioretinopathy mouse model by administration of plasmid DNA

PLVAP plasmid DNA comprising CMV and CD144 promoters was subretinally injected into the diabetic chorioretinopathy model of Example 5-1 from 16 weeks of age in the same manner as in the preparation and injection methods of the solution in Example 2-2, and the structure of choroidal capillaries was confirmed four weeks later.

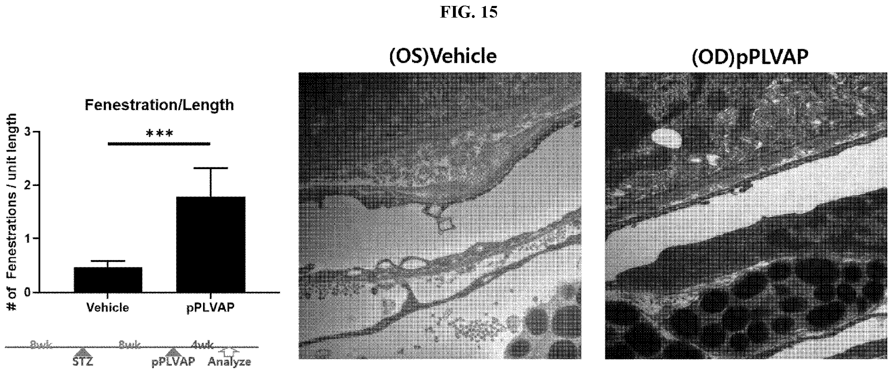

As shown in FIG. 15, the number of fenestrations in the choriocapillary vessel was increased and restored to a normal level in the PLVAP plasmid-administered group, compared to the negative control.

The results show that the PLVAP plasmid DNA of the present invention normalized age-related macular degeneration as well as the structure of a choriocapillary vessel in the mice with diabetic chorioretinopathy.

### 5-3. Confirmation of inhibitory effect of administration of plasmid DNA on onset and progression of diabetic chorioretinopathy

In the diabetic chorioretinopathy model of Example 5-1, the PLVAP plasmid was subretinally injected into 16-week-old mice in the same manner as in the preparation and injection of the PLVAP plasmid solution used in Example 2-2. The progression of retinal degeneration was analyzed by fundus photography and choroid angiography.

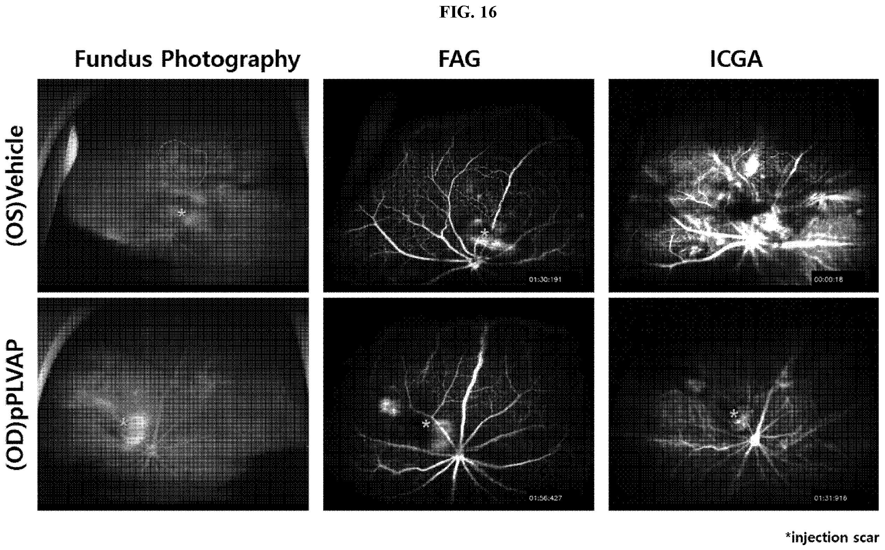

As shown in FIG. 16, it was confirmed from the results of fundus photography that the expansion of and leakage from a choroid vessel were observed at the same position as cotton wool spots in the retina of the control (a red dotted line area). Also, it was confirmed that choroidal hyperfluorescent spots indicating inflammation in the choroidal tissue and a retinal microaneurysm, which are representative medical features of diabetic retinopathy, were also observed at various sites. On the contrary, it was confirmed that the cotton wool spots were not observed in the fundus photography in the case of the experimental group injected with the PLVAP plasmid. Also, it was confirmed from the ICGA results that the expansion of and leakage from the choroid vessel were not found, and the microaneurysms or hyperfluorescent spots were significantly reduced.

Therefore, it was confirmed that the subretinal administration of the PLVAP plasmid was effective in preventing and treating diabetic retinopathy and diabetic chorioretinopathy.

### 5-4. Confirmation of effect of PLVAP plasmid DNA on restoration of impaired visual function

The PLVAP plasmid was subretinally injected into the diabetic chorioretinopathy model used in Example 5-1 from 16 weeks of age. An electroretinogram (ERG) was performed to evaluate the visual function of the diabetic chorioretinopathy model.

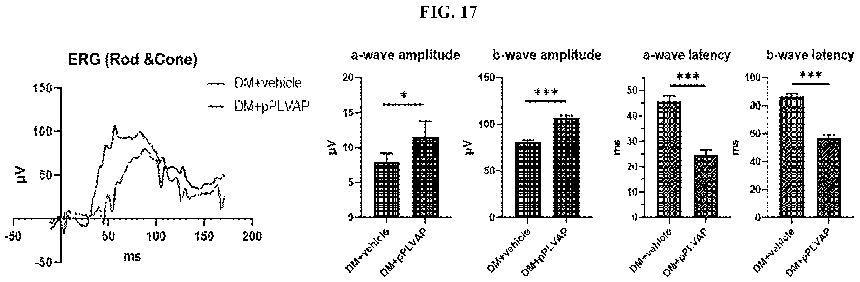

As shown in FIG. 17, it was confirmed from the results of visual function analysis that the amplitudes of a and b waves increased and the latency decreased to restore the function of visual cells in the experimental group into which the PLVAP plasmid was subretinally administered in the diabetic retinopathy/diabetic chorioretinopathy model, compared to the control.

This indicates that the PLVAP plasmid DNA of the present invention was effective in restoring the impaired visual function caused by diabetic retinopathy/chorioretinopathy.

### Example 6. Confirmation of effect of mammalian plasmid vector expressing PLVAP using CMV promoter

### 6-1. Construction of mammalian plasmid vector expressing PLVAP

As shown in FIG. 18, a DNA sequence capable of expressing a PLVAP protein, a CMV promoter as a mammalian co-expression promoter, and an integrating linker attB1/2 were inserted to construct a plasmid DNA having the total size of approximately 7,500 bp. A method of constructing such a plasmid was as described above in Example 1-1.

### 6-2. Confirmation of therapeutic effect of PLVAP-expressing mammalian plasmid vector on age-related macular degeneration

The details of delivering the PLVAP plasmid DNA of Example 6-1 in vivo were used in the same manner as in Example 2-2. 6 weeks after injection, the structure of choriocapillary vessel was checked. The leakage from and expansion of choroid vessel were analyzed by choroid angiography.

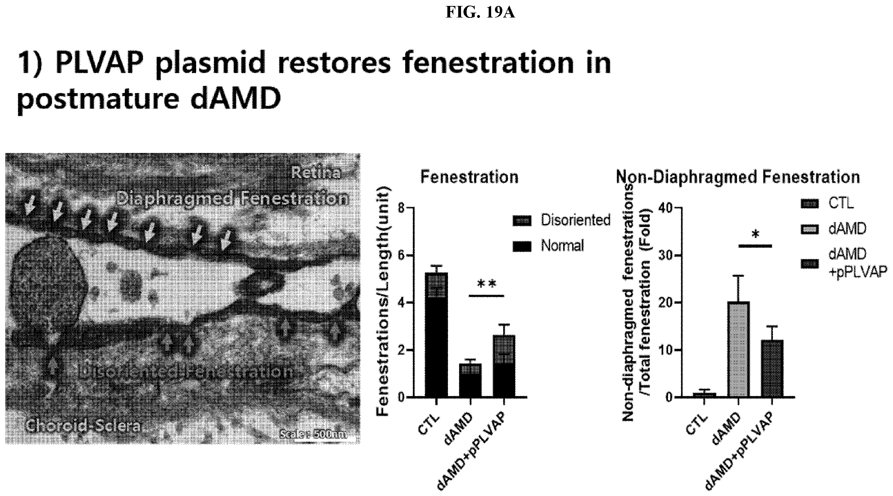

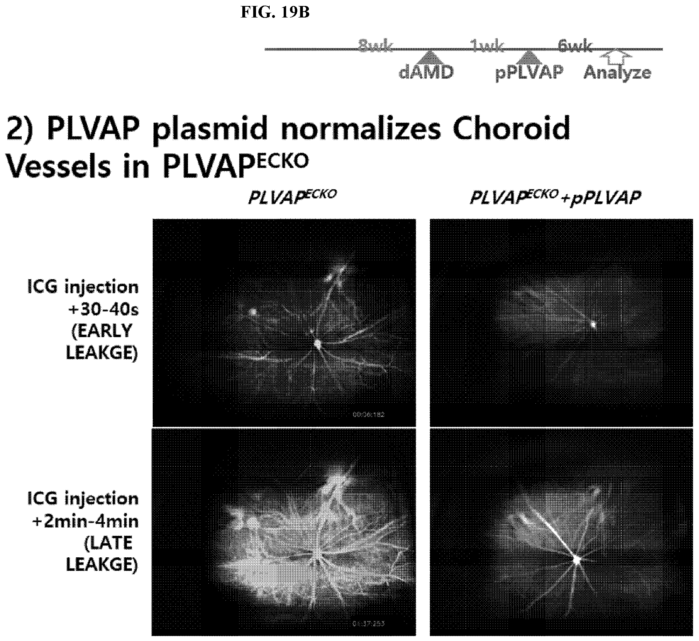

As shown in FIGS. 19A and 19B, it was confirmed that, at one month after the PLVAP plasmid DNA was injected using CMV as the promoter, the number of fenestrations in the choroid vessel increased and the polarity was normalized toward the retina by supplementation of the PLVAP, and retinal waste was well discharged. Also, it was confirmed that the number of the diaphragm-free fenestrations decreased by supplementation of the PLVAP. In addition, it was confirmed that the leakage from and expansion of the choroid vessel in the age-related macular degeneration model were remarkably reduced after the injection of PLVAP plasmid DNA.

Therefore, it was revealed that the CMVpromoter-PLVAP had a therapeutic effect of improving vision because the symptoms of retinal degeneration were improved by administration of CMVpromoter-PLVAP.

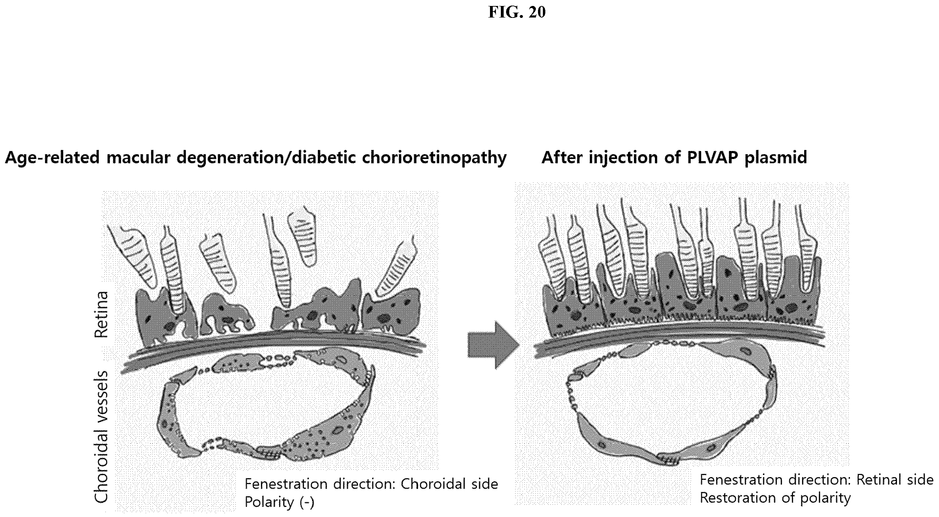

Also, as shown in FIG. 20, it can be seen that the ultrastructure of choroid vessel was restored by injection of the PLVAP plasmid.

### Example 7. Confirmation of expression of PLVAP in aging and diabetic patients

Expression of PLVAP in elderly and diabetic patients was examined by checking the medical records on underlying diseases in donor eyeballs, and the eyeballs obtained from the patients were used. Only the eyeballs from donors having no ophthalmic and metabolic underlying diseases were used, and the patients were divided into Young aged 20 to 40 years old and Old aged 60 or more years old, depending on age. An experiment of confirming a direction of fenestrations was performed only on the Young group. The eyeballs donated from the diabetic patients were not separately divided according to age, but the eyeballs donated from the age groups corresponding to all the patients aged more than 40 and less than 70 years old were used as the control. The data from the patients participating in the study was used as the ultra-widefield fundus photography and OCT photography results of the diabetic patients.

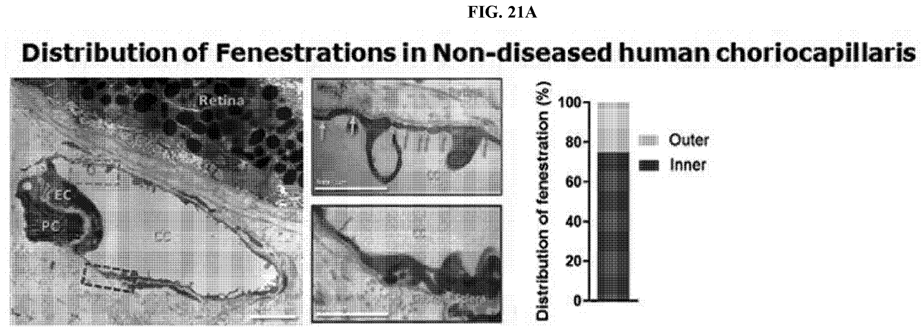

As shown in FIG. 21A, it can be seen that the fenestrations were uniformly distributed in a normal human choriocapillary vessel.

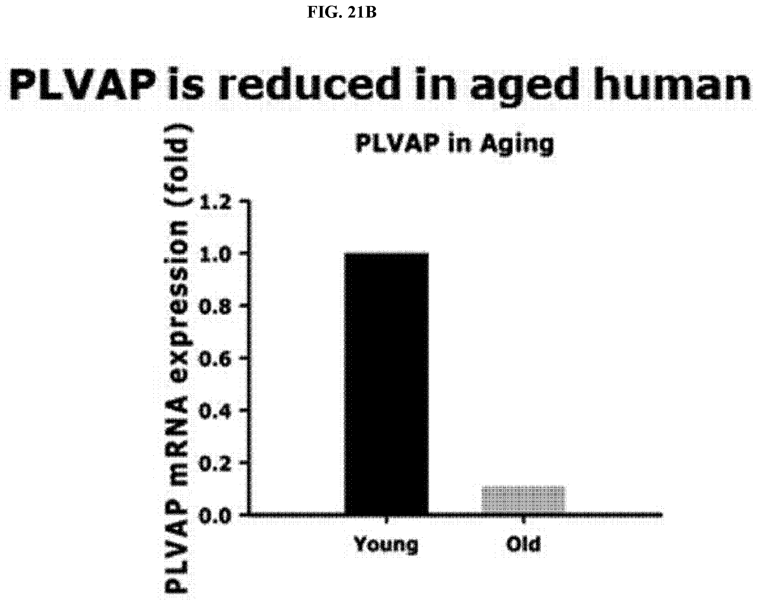

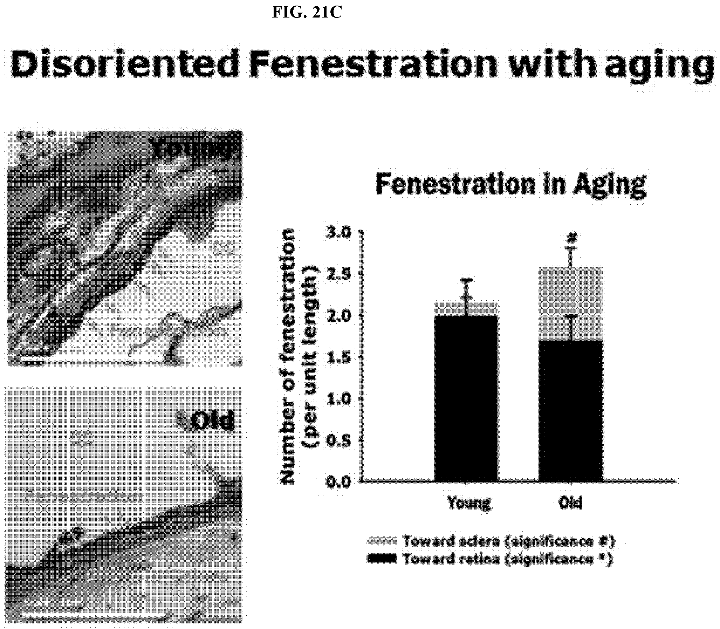

Also, as shown in FIGS. 21B and 21C, it was confirmed that the absolute number of fenestrations decreased as the PLVAP expression was reduced in choriocapillary endothelial cells in the elderly people, and a change in polarity of fenestrations in the capillary vessel was caused as the fenestrations were directed toward the sclera rather than the retina, which is the opposite direction. Due to this change in the ultrastructure, it was confirmed that waste was not excreted from the retina but was accumulated in the retina to cause inflammation, and macular degeneration was caused by the impaired retinal function.

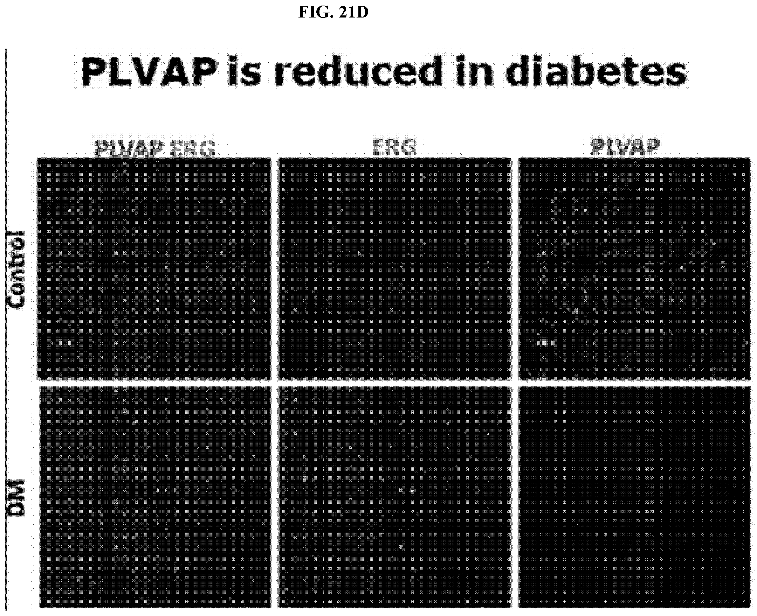

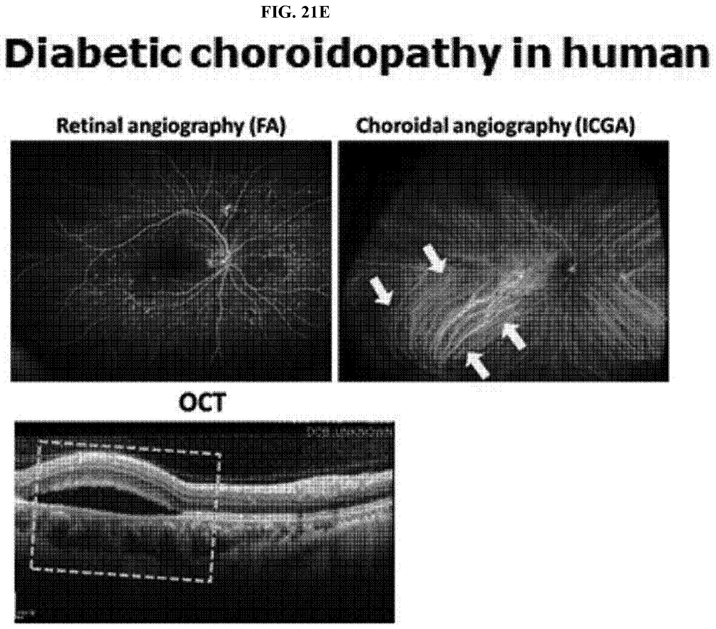

In addition, as shown in FIGS. 21D and 21E, it was confirmed that the PLVAP expression in the choriocapillary endothelial cells of the diabetic patients was reduced.

The results support that the PLVAP supplementation of the choroid vessel reduced due to aging and diabetes showed a therapeutic effect on retinal diseases including age-related macular degeneration and diabetic chorioretinopathy.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described examples are only illustrative in all aspects and not restrictive.

### [Industrial Applicability]

On the basis that a reduction in PLVAP may lead to retinal degeneration by causing a change in the ultra-microstructure of choroidal vessels, the present inventors confirmed that the administration of PLVAP plasmid DNA to an animal model of a retinal or choroidal disease had the effect of increasing fenestrations, which is important for maintaining the normal function of choroidal vessels, while maintaining the structure and function of choroidal vessels. Therefore, the PLVAP protein or gene can be usefully used for the treatment of a retinal or choroidal disease, and thus has industrial applicability.

## Claims

1. A pharmaceutical composition for preventing or treating a retinal or choroidal disease, the pharmaceutical composition comprising a plasmalemma vesicle-associated protein (PLVAP) protein, an expression or activity activator of the protein, or a nucleotide sequence encoding the PLVAP protein, as an active ingredient.

2. The pharmaceutical composition of claim 1, wherein the PLVAP protein comprises an amino acid sequence represented by SEQ ID NO: 25, or an amino acid sequence having a sequence homology of 80% or more therewith.

3. The pharmaceutical composition of claim 1, wherein the PVLAP protein is a PLVAP protein, a fragment thereof, a variant thereof, or a fusion protein thereof.

4. The pharmaceutical composition of claim 1, wherein the nucleotide sequence encoding the PLVAP protein is comprised in a recombinant vector.

5. The pharmaceutical composition of claim 4, wherein the recombinant vector comprises a promoter, an enhancer, an intron, a reporter marker sequence, a protein purification tag, a termination sequence, an mRNA stabilization sequence, an exogenous gene sequence, a cell selection marker sequence, or a combination thereof.

6. The pharmaceutical composition of claim 4, wherein the recombinant vector comprises a promoter that induces specific expression in vascular endothelial cells, and the promoter is one or more selected from the group consisting of CD144, TIE1, FLT1, and EMCN.

7. The pharmaceutical composition of claim 1, wherein the retinal or choroidal disease is a retinal or choroidal disease associated with vasopermeability.

8. The pharmaceutical composition of claim 1, wherein the retinal or choroidal disease is retinitis pigmentosa (RP), Leber congenital amaurosis (LCA), Stargardt disease, Usher's syndrome, choroideremia, rod-cone or cone-rod dystrophy, ciliopathy, a mitochondrial disorder, progressive retinal atrophy, a degenerative retinal disease, age-related macular degeneration (AMD), wet AMD, dry AMD, central serous chorioretinopathy, the pachychoroid disease spectrum, degenerative myopia, nodular choroidopathy, chorioretinitis, choroidal tumors, choroidal neovascularization, hereditary choroidal disease, geographic atrophy, familial or acquired maculopathy, a retinal photoreceptor disease, a retinal pigment epithelial-based disease, diabetic retinopathy, diabetic chorioretinopathy, cystoid macular edema, uveitis, retinal detachment, traumatic retinal injury, iatrogenic retinal injury, macular holes, macular telangiectasia, a ganglion cell disease, an optic nerve cell disease, glaucoma, optic neuropathy, an ischemic retinal disease, retinopathy of prematurity, retinal vascular occlusion, a familial retinal arterial macroaneurysm, a retinal vascular disease, an ocular vascular disease, retinal nerve cell degeneration retinal nerve cell degeneration due to glaucoma, ischemic optic neuropathy, or a combination thereof.

9. The pharmaceutical composition of claim 1, wherein the nucleotide sequence comprises a base sequence represented by SEQ ID NO: 1, or a base sequence having a sequence homology of 80% or more therewith.

10. The pharmaceutical composition of claim 1, wherein the pharmaceutical composition is formulated for administering to a subject via one or more routes selected from the group consisting of oral, subcutaneous, intraperitoneal, intrapulmonary, intranasal, intramuscular, intravenous, intraarterial and topical ocular administration.

11. The pharmaceutical composition of claim 10, wherein the topical ocular administration is intraconjunctival administration, intravitreal administration, subretinal administration, suprachoroidal administration, subconjunctival administration, administration under the Tenon's capsule, or a combination thereof.

12. The pharmaceutical composition of claim 1, wherein the pharmaceutical composition further comprises one or more selected from the group consisting of pharmaceutically acceptable carriers, excipients and dilulents.

13. The pharmaceutical composition of claim 1, wherein the pharmaceutical composition restores the fenestrations of endothelial cells.

14. The pharmaceutical composition of claim 1, wherein the pharmaceutical composition further comprises an anti-VEGF agent.

15. A quasi-drug composition for the prevention or treatment of a retinal or choroidal disease, the quasi-drug composition comprising a plasmalemma vesicle-associated protein (PLVAP) protein, an expression or activity activator of the protein, or a nucleotide sequence encoding the PLVAP protein, as an active ingredient.

16. A method of preventing, ameliorating or treating a retinal or choroidal disease, the method comprising: administering a pharmaceutical composition comprising a plasmalemma vesicle-associated protein (PLVAP) protein, an expression or activity activator of the protein, or a nucleotide sequence encoding the PLVAP protein, as an active ingredient, to a subject.

17. The method of claim 16, wherein the nucleotide sequence comprises a base sequence represented by SEQ ID NO: 1, or a base sequence having a sequence homology of 80% or more therewith.

18. The method of claim 16, wherein the pharmaceutical composition is administered to a subject via one or more routes selected from the group consisting of oral, subcutaneous, intraperitoneal, intrapulmonary, intranasal, intramuscular, intravenous, intraarterial and topical ocular administration.

19. The method of claim 16, wherein the subject is a subject in need of treatment of a retinal or choroidal disease, and
wherein the retinal or choroidal disease is retinitis pigmentosa (RP), Leber congenital amaurosis (LCA), Stargardt disease, Usher's syndrome, choroideremia, rod-cone or cone-rod dystrophy, ciliopathy, a mitochondrial disorder, progressive retinal atrophy, a degenerative retinal disease, age-related macular degeneration (AMD), wet AMD, dry AMD, central serous chorioretinopathy, the pachychoroid disease spectrum, degenerative myopia, nodular choroidopathy, chorioretinitis, choroidal tumors, choroidal neovascularization, hereditary choroidal disease, geographic atrophy, familial or acquired maculopathy, a retinal photoreceptor disease, a retinal pigment epithelial-based disease, diabetic retinopathy, diabetic chorioretinopathy, cystoid macular edema, uveitis, retinal detachment, traumatic retinal injury, iatrogenic retinal injury, macular holes, macular telangiectasia, a ganglion cell disease, an optic nerve cell disease, glaucoma, optic neuropathy, an ischemic retinal disease, retinopathy of prematurity, retinal vascular occlusion, a familial retinal arterial macroaneurysm, a retinal vascular disease, an ocular vascular disease, retinal nerve cell degeneration retinal nerve cell degeneration due to glaucoma, ischemic optic neuropathy, or a combination thereof.

20. The method of claim 16, wherein the pharmaceutical composition is administered simultaneously or sequentially with an anti-VEGF agent.

21. A use of a pharmaceutical composition comprising a plasmalemma vesicle-associated protein (PLVAP) protein, an expression or activity activator of the protein, or a nucleotide sequence encoding the PLVAP protein, as an active ingredient, for preventing, ameliorating or treating a retinal or choroidal disease.

22. A use of a plasmalemma vesicle-associated protein (PLVAP) protein, an expression or activity activator of the protein, or a nucleotide sequence encoding the PLVAP protein for producing a drug for preventing or treating a retinal or choroidal disease.

23. A plasmalemma vesicle-associated protein (PLVAP) protein, an expression or activity activator of the protein, or an isolated nucleotide sequence encoding the PLVAP protein, which is **characterized by** improving the ultra-microstructure of choroidal vessels.

24. A pharmaceutical composition for improving an ultra-microstructure of choroidal vessels, the pharmaceutical composition comprising a plasmalemma vesicle-associated protein (PLVAP) protein, an expression or activity activator of the protein, or an isolated nucleotide sequence encoding the PLVAP protein, as an active ingredient.

25. A method of improving an ultra-microstructure of choroidal vessels, the method comprising: administering a composition comprising a plasmalemma vesicle-associated protein (PLVAP) protein, an expression or activity activator of the protein, or an isolated nucleotide sequence encoding the PLVAP protein, as an active ingredient, to a subject.

26. A use of a composition comprising a plasmalemma vesicle-associated protein (PLVAP) protein, an expression or activity activator of the protein, or an isolated nucleotide sequence encoding the PLVAP protein, as an active ingredient, for improving an ultra-microstructure of choroidal vessels.
